# EUROPEAN PATENT SPECIFICATION

(11) **EP 3 080 275 B1**
(45) Date of publication and mention of the grant of the patent: **15.01.2020**
(21) Application number: 14812223.7
(22) Date of filing: 12.12.2014
(51) Int. Cl.: C12N 15/79, C12N 15/82, C12N 15/90, C12N 15/63

(54) **METHOD OF SELECTION OF TRANSFORMED DIATOMS USING NUCLEASE**
VERFAHREN ZUR AUSWAHL VON MITTELS NUKLEASE TRANSFORMIERTEN DIATOMEEN
PROCÉDÉ DE SÉLECTION DE DIATOMÉES TRANSFORMÉES À L'AIDE DE NUCLÉASE

(30) Priority: 13.12.2013 DK 201370773
(43) Date of publication of application: 19.10.2016
(73) Proprietor: Cellectis, 75013 Paris (FR)
(72) Inventor: DUCHATEAU, Philippe, F-91210 Draveil (FR); DABOUSSI, Fayza, 31750 ESCALQUENS (FR)
(74) Representative: Santarelli
(86) International application number: PCT/EP2014/077513
(87) International publication number: WO 2015/086798

(56) References cited:
- WO-A2-2012/017329
- NELSON JULIE A E ET AL: "Targeted disruption of the NIT8 gene in Chlamydomonas reinhardtii", MOLECULAR AND CELLULAR BIOLOGY, vol. 15, no. 10, 1995, pages 5762-5769, XP002728707, ISSN: 0270-7306
- SAKAGUCHI TOSHIRO ET AL: "Identification of the UMP Synthase Gene by Establishment of Uracil Auxotrophic Mutants and the Phenotypic Complementation System in the Marine Diatom Phaeodactylum tricornutum", PLANT PHYSIOLOGY (ROCKVILLE), vol. 156, no. 1, May 2011 (2011-05), pages 78-89, XP002728708, ISSN: 0032-0889
- PALOMBELLA ANTHONY L ET AL: "Identification of the gene encoding the tryptophan synthase beta-subunit from Chlamydomonas reinhardtii", PLANT PHYSIOLOGY (ROCKVILLE), vol. 117, no. 2, June 1998 (1998-06), pages 455-464, XP002728709, ISSN: 0032-0889
- ROHR JENNIFER ET AL: "Tandem inverted repeat system for selection of effective transgenic RNAi strains in Chlamydomonas", PLANT JOURNAL, vol. 40, no. 4, November 2004 (2004-11), pages 611-621, XP002728710, ISSN: 0960-7412
- FAYZA DABOUSSI ET AL: "Genome engineering empowers the diatom Phaeodactylum tricornutum for biotechnology", NATURE COMMUNICATIONS, vol. 5, 29 May 2014 (2014-05-29), XP055128955, DOI: 10.1038/ncomms4831

### SEQUENCE LISTING

<110> CELLECTIS
<120> NEW METHOD OF SELECTION OF ALGAL-TRANSFORMED CELLS USING NUCLEASE
<130> P81314602PCT00
<150> PA201370773
   <151> 2013-12-13
<160> 17
<170> PatentIn version 3.5
<210> 1
   <211> 1557
   <212> DNA
   <213> Phaeodactylum tricornutum
<400> 1
   atggccaccc cctcttttcg atcaaagctt gaagctcgag tcgccgcagt caactctctc      60
   ttgtgcgttg gtctagaccc gcacgagaaa gagctgtttg cagacggatg ggaaggcgtg     120
   ccggaagaaa atcgctgtga cgcggccttt accttttgca aaacgttggt cgacgcaaca     180
   ttgccttaca cggcctgcta caaacccaat gctgcctttt tcgaggcgtt aggcgatgga     240
   gggatggcgg ttctgcgacg agtttgtcaa aacataatac cggatgatgt gccgattttg     300
   ttggatgtca agcgcggcga cattggctcg accgctgcgg cctacgccga agcgtgctat     360
   ggtttgggtg cagactgtgt cacgctttca ccactgatgg gatgggactc agtcagtccc     420
   tttgttacag aaaagtacgt tcacaaagga gcatttttgc tgtgcaaaac gtcaaatcct     480
   ggatccaacg attttttagc tctgggatta cgttcaaatg aatgtttata cgaaagaatt     540
   gccaagcttg ttggctcgga atgggctcag cagaccgaga gttcattggg actcgttgtc     600
   ggggccacag atccagtggc cttgtccaaa gcgagaaagg ctgcaggcga cgacacctgg     660
   attctagcac ccggcgttgg tgctcaaggt ggagatcttc tagaagcagc gcaggctgga     720
   ttgaatacaa aggggacttg catgctaatt cccgtgtcta ggggtatcag caaagctacg     780
   gacccagcgc aggctgcaaa agaattgcag gagaggattc agaaagctcg ggaccaagtc     840
   gtggccgcac acatgataaa aaagagttca gacgaagata ttaaactcta tcaacgcgag     900
   tttcttgaat ttagtctgtc tcaaggtgtt ctcaaattcg gctcttttgt gctgaaaagc     960
   ggccgcacct ctccatattt tttcaacgcc ggtctttttg cttctggcgc tgcgttaagc    1020
   aagcttggga aagcctatgc ttcgactatc atgtcctcgg aattattagc tgctgggccc    1080
   aaccaagtca attttgatgt gatttttggt cctgcataca agggtatttc tctaggtgct    1140
   gtcgttggaa gcgctctgta taacgatttt gaagtagatg tcggttttgc gtatgaccga    1200
   aaagaggcaa aggatcatgg ggaaggtggt aaattggtcg ggacttcgtt ggaaggaaaa    1260
   cgagttctga ttgtagatga cgtaatcaca gcgggaaccg ccattcgtga gtcgcacact    1320
   ttgctcaacg atgtgggtgc tttgccagtt ggagtagtta ttgccctcga tcgagccgaa    1380
   attcgctcta tggaggacaa gatttccgct gttcaagcag tcgcacgaga tctatctctt    1440
   ctggtcgtgt caattgtcag tcttcctcaa ctacagacgt ttctcgaacg aagtccggac    1500
   tacggcgatg aaacgctgga aaaagtaact aagtatcgaa acgaatacgg agtgtaa        1557
<210> 2
   <211> 2733
   <212> DNA
   <213> Phaeodactylum tricornutum
<220>
   <221> misc_feature
   <222> (2619)..(2619)
   <223> n is a, c, g, or t
<400> 2
   atggtaccga aacctgaaga tcccacagtc aaggcagaga acaatgcggc gatggatcaa 60
   cttagtctcc tcgacaaaga agatatatcg tcggcttctc gctcgtgccg agaactctac 120
   ggaccttacc ccaaagctat tcctgtgccg ttcttgaatt ctcgtaacga agctcgcgaa 180
   ggtgacactc ccgccgccag cgtcatcgcg caagccaaaa ccatctttga cgtaccggcg 240
   gactatcgtg acgtgggaac accggatgaa tgggttcccc gcgatggacg cctcgtgcgt 300
   ctgacgggta agcatcccct caacgtcgaa ccaccgctgg cgattctgaa gcagcatcga 360
   tttattacgc cgtcctcgtt gcattacgta cgcaaccatg gagcgtgccc gaagctgtct 420
   tggaaacaac acactgtttg tgtgggagga aaactggtac cgaatgcctt ggagctctcg 480
   atggacgaaa tcgtagcgat ggaaccgcga gagctgcccg tcacgttggt ctgtgccgga 540
   aatcgtcgga aggaacaaaa catgatccgt caaacaatcg gcttcaactg gggcccgagc 600
   ggcgtctcaa ccagcgtttg gaagggagtg ctcctacgcg atttgttgct ccgcgcaggg 660
   gtttcggaaa agaacatggc agggaagcac gtcgaattta ttggtgtcga agacttgccg 720
   aacaaggtgg gacccgggcc gttccaggag gaaccatggg gcaaacttgt caagtacgga 780
   accagtgtcc cgctcgctcg ggctatgaat ccagcgtacg acatcctcat tgcctatgag 840
   cagaacggcg aagtcttgca gcccgatcac gggtaccccg tccgtctcat cattcctggt 900
   tatattggag gacggatgat taaatggctt aaatacatca acgtgattcc gcacgaaccc 960
   aagaatcact atcattacca cgacaatcgc attttacagg gaggttggtg gtacaaaccg 1020
   gagtacattt tcaatgaact caacatcaat tcggccatcg cggctcctga tcacaatgaa 1080
   acgctttcga tcgccaagaa tattgccaag acgtatgacg ttacgggtta cgcatatact 1140
   ggtggtggtc gtctcatcac cagggtcgaa atttcagttg atggcggtat ccattgggaa 1200
   cttgccaaac gtgaacgcaa ggagcagcca acggactacg gaatgtactg gtgctggact 1260
   tggtggaact acgaagtaaa ggtggccgac ttggtgggag ccaaggaaat tatatgccgc 1320
   gcctgggatg agtccaacaa ccctcagcca gttgttccaa catggaatct gatgggtatg 1380
   gggaataatc aagcctttcg tgtcaaggta cacatggaca agacagctag cggcgagcat 1440
   gtgtttcggt ttgagcatcc aactcagcct ggtcaacaaa ctggtgggtg gatgacaaag 1500
   gtcgccacca agcctgagtc ggccgggttc ggacggttgc tggaagtgca ggctgagtcc 1560
   aaagaagacg cggccccggc tccacctccg aaggaaaata ccaaaatttt cacgatggaa 1620
   gagattgaaa agcacaacac tgaagaagac tgttggattg tggtgaagga tcgtgtctac 1680
   gactgtaccg agtatctaga gctgcaccct ggcggcattg actcgattgt tatcaacggc 1740
   ggcgcagatt ccacggaaga ctttgtggca atccactcta ccaaggctac aaagatgctc 1800
   gagaagtact acattggcca gctcgacaaa agtagtgtgg ccgaggagaa aaaacaagaa 1860
   gacgaacctc tcgtcgatgc cgatggcaat gctcttgcct tgaacccaaa gaagaagacg 1920
   ccatttcgtc tccaaaacaa aatcacactt agtcgagaca gctacctatt ggattttgct 1980
   ttgccaagcc caaagcatgt tttggggcta cccacgggaa agcacatgtt tatttcggcc 2040
   ctcattaatg gagagatggt actccggcgc tacactccta tctcatccaa ttacgacatt 2100
   ggatgtgtaa agtttgttgt caaggcatac cgtccgtgtg aacgctttcc agacggtggc 2160
   aagatgagcc aatacctaga ccagatcaat gttggcgact atgttgatat gcgcggacca 2220
   gttggggaat ttgagtactc ggccaacggc agttttacaa tcgacgccga accttgtttt 2280
   gccaccaggt tcaacatgct tgctgggggg accggcataa cgcccgtaat gcagattgct 2340
   gcggaaattt tgcgaaaccc acaagaccct acacaaatgt cccttatttt tgcatgccgc 2400
   gaggaaggcg atctcttgat gcgaagcact ttggacgaat gggctgctaa ctttcctgac 2460
   aagttcaaga ttcactacat cctatctgac agctggtctt ccgactggaa gtattccaca 2520
   ggattcgtag acaaagcgct attttccgag tacttgtacg aagcaggcga taatgtttac 2580
   agcctcatgt gcggcccacc aattatgtta gagaaaggnt gccgtccaaa cttgggagag 2640
   ccttggtcac aaaaaggaca aaattttttc cttttaaaag ttcttggact gattgtcata 2700
   tcaattttgc actttacaat acattttcaa tag 2733
<210> 3
   <211> 49
   <212> DNA
   <213> Phaeodactylum tricornutum
<400> 3
   tttagtctgt ctctaggtgt tctcaaattc ggctcttttg tgctgaaaa 49
<210> 4
   <211> 49
   <212> DNA
   <213> Phaeodactylum tricornutum
<400> 4
   tgaagcagca tcgatttatt acgccgtcct cgttgcatta cgtacgcaa 49
<210> 5
   <211> 1088
   <212> PRT
   <213> artificial sequence
<220>
   <223> UMPS-TALEN-T01-L1
<400> 5
   Met Gly Asp Pro Lys Lys Lys Arg Lys Val Ile Asp Tyr Pro Tyr Asp
   1               5                   10                  15
   Val Pro Asp Tyr Ala Ile Asp Ile Ala Asp Pro Ile Arg Ser Arg Thr
               20                  25                  30
   Pro Ser Pro Ala Arg Glu Leu Leu Pro Gly Pro Gln Pro Asp Gly Val
           35                  40                  45
   Gln Pro Thr Ala Asp Arg Gly Val Ser Pro Pro Ala Gly Gly Pro Leu
       50                  55                  60
   Asp Gly Leu Pro Ala Arg Arg Thr Met Ser Arg Thr Arg Leu Pro Ser
   65                  70                  75                  80
   Pro Pro Ala Pro Ser Pro Ala Phe Ser Ala Gly Ser Phe Ser Asp Leu
                   85                  90                  95
   Leu Arg Gln Phe Asp Pro Ser Leu Phe Asn Thr Ser Leu Phe Asp Ser
               100                 105                 110
   Leu Pro Pro Phe Gly Ala His His Thr Glu Ala Ala Thr Gly Glu Trp
           115                 120                 125
   Asp Glu Val Gln Ser Gly Leu Arg Ala Ala Asp Ala Pro Pro Pro Thr
       130                 135                 140
   Met Arg Val Ala Val Thr Ala Ala Arg Pro Pro Arg Ala Lys Pro Ala
   145                 150                 155                 160
   Pro Arg Arg Arg Ala Ala Gln Pro Ser Asp Ala Ser Pro Ala Ala Gln
                   165                 170                 175
   Val Asp Leu Arg Thr Leu Gly Tyr Ser Gln Gln Gln Gln Glu Lys Ile
               180                 185                 190
   Lys Pro Lys Val Arg Ser Thr Val Ala Gln His His Glu Ala Leu Val
           195                 200                 205
   Gly His Gly Phe Thr His Ala His Ile Val Ala Leu Ser Gln His Pro
       210                 215                 220
   Ala Ala Leu Gly Thr Val Ala Val Lys Tyr Gln Asp Met Ile Ala Ala
   225                 230                 235                 240
   Leu Pro Glu Ala Thr His Glu Ala Ile Val Gly Val Gly Lys Gln Trp
                   245                 250                 255
   Ser Gly Ala Arg Ala Leu Glu Ala Leu Leu Thr Val Ala Gly Glu Leu
               260                 265                 270
   Arg Gly Pro Pro Leu Gln Leu Asp Thr Gly Gln Leu Leu Lys Ile Ala
           275                 280                 285
   Lys Arg Gly Gly Val Thr Ala Val Glu Ala Val His Ala Trp Arg Asn
       290                 295                 300
   Ala Leu Thr Gly Ala Pro Leu Asn Leu Thr Pro Gln Gln Val Val Ala
   305                 310                 315                 320
   Ile Ala Ser Asn Gly Gly Gly Lys Gln Ala Leu Glu Thr Val Gln Arg
                   325                 330                 335
   Leu Leu Pro Val Leu Cys Gln Ala His Gly Leu Thr Pro Gln Gln Val
               340                 345                 350
   Val Ala Ile Ala Ser Asn Gly Gly Gly Lys Gln Ala Leu Glu Thr Val
           355                 360                 365
   Gln Arg Leu Leu Pro Val Leu Cys Gln Ala His Gly Leu Thr Pro Glu
       370                 375                 380
   Gln Val Val Ala Ile Ala Ser Asn Ile Gly Gly Lys Gln Ala Leu Glu
   385                 390                 395                 400
   Thr Val Gln Ala Leu Leu Pro Val Leu Cys Gln Ala His Gly Leu Thr
                   405                 410                 415
   Pro Gln Gln Val Val Ala Ile Ala Ser Asn Asn Gly Gly Lys Gln Ala
               420                 425                 430
   Leu Glu Thr Val Gln Arg Leu Leu Pro Val Leu Cys Gln Ala His Gly
           435                 440                 445
   Leu Thr Pro Gln Gln Val Val Ala Ile Ala Ser Asn Gly Gly Gly Lys
       450                 455                 460
   Gln Ala Leu Glu Thr Val Gln Arg Leu Leu Pro Val Leu Cys Gln Ala
   465                 470                 475                 480
   His Gly Leu Thr Pro Glu Gln Val Val Ala Ile Ala Ser His Asp Gly
                   485                 490                 495
   Gly Lys Gln Ala Leu Glu Thr Val Gln Arg Leu Leu Pro Val Leu Cys
               500                 505                 510
   Gln Ala His Gly Leu Thr Pro Gln Gln Val Val Ala Ile Ala Ser Asn
           515                 520                 525
   Gly Gly Gly Lys Gln Ala Leu Glu Thr Val Gln Arg Leu Leu Pro Val
       530                 535                 540
   Leu Cys Gln Ala His Gly Leu Thr Pro Gln Gln Val Val Ala Ile Ala
   545                 550                 555                 560
   Ser Asn Asn Gly Gly Lys Gln Ala Leu Glu Thr Val Gln Arg Leu Leu
                   565                 570                 575
   Pro Val Leu Cys Gln Ala His Gly Leu Thr Pro Gln Gln Val Val Ala
               580                 585                 590
   Ile Ala Ser Asn Gly Gly Gly Lys Gln Ala Leu Glu Thr Val Gln Arg
           595                 600                 605
   Leu Leu Pro Val Leu Cys Gln Ala His Gly Leu Thr Pro Glu Gln Val
       610                 615                 620
   Val Ala Ile Ala Ser His Asp Gly Gly Lys Gln Ala Leu Glu Thr Val
   625                 630                 635                 640
   Gln Arg Leu Leu Pro Val Leu Cys Gln Ala His Gly Leu Thr Pro Gln
                   645                 650                 655
   Gln Val Val Ala Ile Ala Ser Asn Gly Gly Gly Lys Gln Ala Leu Glu
               660                 665                 670
   Thr Val Gln Arg Leu Leu Pro Val Leu Cys Gln Ala His Gly Leu Thr
           675                 680                 685
   Pro Glu Gln Val Val Ala Ile Ala Ser His Asp Gly Gly Lys Gln Ala
       690                 695                 700
   Leu Glu Thr Val Gln Arg Leu Leu Pro Val Leu Cys Gln Ala His Gly
   705                 710                 715                 720
   Leu Thr Pro Gln Gln Val Val Ala Ile Ala Ser Asn Gly Gly Gly Lys
                   725                 730                 735
   Gln Ala Leu Glu Thr Val Gln Arg Leu Leu Pro Val Leu Cys Gln Ala
               740                 745                 750
   His Gly Leu Thr Pro Glu Gln Val Val Ala Ile Ala Ser Asn Ile Gly
           755                 760                 765
   Gly Lys Gln Ala Leu Glu Thr Val Gln Ala Leu Leu Pro Val Leu Cys
       770                 775                 780
   Gln Ala His Gly Leu Thr Pro Gln Gln Val Val Ala Ile Ala Ser Asn
   785                 790                 795                 800
   Asn Gly Gly Lys Gln Ala Leu Glu Thr Val Gln Arg Leu Leu Pro Val
                   805                 810                 815
   Leu Cys Gln Ala His Gly Leu Thr Pro Gln Gln Val Val Ala Ile Ala
               820                 825                 830
   Ser Asn Gly Gly Gly Arg Pro Ala Leu Glu Ser Ile Val Ala Gln Leu
           835                 840                 845
   Ser Arg Pro Asp Pro Ala Leu Ala Ala Leu Thr Asn Asp His Leu Val
       850                 855                 860
   Ala Leu Ala Cys Leu Gly Gly Arg Pro Ala Leu Asp Ala Val Lys Lys
   865                 870                 875                 880
   Gly Leu Gly Asp Pro Ile Ser Arg Ser Gln Leu Val Lys Ser Glu Leu
                   885                 890                 895
   Glu Glu Lys Lys Ser Glu Leu Arg His Lys Leu Lys Tyr Val Pro His
               900                 905                 910
   Glu Tyr Ile Glu Leu Ile Glu Ile Ala Arg Asn Ser Thr Gln Asp Arg
           915                 920                 925
   Ile Leu Glu Met Lys Val Met Glu Phe Phe Met Lys Val Tyr Gly Tyr
       930                 935                 940
   Arg Gly Lys His Leu Gly Gly Ser Arg Lys Pro Asp Gly Ala Ile Tyr
   945 950 955 960
   Thr Val Gly Ser Pro Ile Asp Tyr Gly Val Ile Val Asp Thr Lys Ala
   965 970 975
   Tyr Ser Gly Gly Tyr Asn Leu Pro Ile Gly Gln Ala Asp Glu Met Gln
   980 985 990
   Arg Tyr Val Glu Glu Asn Gln Thr Arg Asn Lys His Ile Asn Pro Asn
   995 1000 1005
   Glu Trp Trp Lys Val Tyr Pro Ser Ser Val Thr Glu Phe Lys Phe
   1010 1015 1020
   Leu Phe Val Ser Gly His Phe Lys Gly Asn Tyr Lys Ala Gln Leu
   1025 1030 1035
   Thr Arg Leu Asn His Ile Thr Asn Cys Asn Gly Ala Val Leu Ser
   1040 1045 1050
   Val Glu Glu Leu Leu Ile Gly Gly Glu Met Ile Lys Ala Gly Thr
   1055 1060 1065
   Leu Thr Leu Glu Glu Val Arg Arg Lys Phe Asn Asn Gly Glu Ile
   1070 1075 1080
   Asn Phe Ala Ala Ala
   1085
<210> 6
   <211> 1094
   <212> PRT
   <213> artificial sequence
<220>
   <223> UMPS-TALEN-T01-R1
<400> 6
   Met Gly Asp Pro Lys Lys Lys Arg Lys Val Ile Asp Lys Glu Thr Ala
   1 5 10 15
   Ala Ala Lys Phe Glu Arg Gln His Met Asp Ser Ile Asp Ile Ala Asp
   20 25 30
   Pro Ile Arg Ser Arg Thr Pro Ser Pro Ala Arg Glu Leu Leu Pro Gly
   35 40 45
   Pro Gln Pro Asp Gly Val Gln Pro Thr Ala Asp Arg Gly Val Ser Pro
       50                  55                  60
   Pro Ala Gly Gly Pro Leu Asp Gly Leu Pro Ala Arg Arg Thr Met Ser
   65                  70                  75                  80
   Arg Thr Arg Leu Pro Ser Pro Pro Ala Pro Ser Pro Ala Phe Ser Ala
                   85                  90                  95
   Gly Ser Phe Ser Asp Leu Leu Arg Gln Phe Asp Pro Ser Leu Phe Asn
               100                 105                 110
   Thr Ser Leu Phe Asp Ser Leu Pro Pro Phe Gly Ala His His Thr Glu
           115                 120                 125
   Ala Ala Thr Gly Glu Trp Asp Glu Val Gln Ser Gly Leu Arg Ala Ala
       130                 135                 140
   Asp Ala Pro Pro Pro Thr Met Arg Val Ala Val Thr Ala Ala Arg Pro
   145                 150                 155                 160
   Pro Arg Ala Lys Pro Ala Pro Arg Arg Arg Ala Ala Gln Pro Ser Asp
                   165                 170                 175
   Ala Ser Pro Ala Ala Gln Val Asp Leu Arg Thr Leu Gly Tyr Ser Gln
               180                 185                 190
   Gln Gln Gln Glu Lys Ile Lys Pro Lys Val Arg Ser Thr Val Ala Gln
           195                 200                 205
   His His Glu Ala Leu Val Gly His Gly Phe Thr His Ala His Ile Val
       210                 215                 220
   Ala Leu Ser Gln His Pro Ala Ala Leu Gly Thr Val Ala Val Lys Tyr
   225                 230                 235                 240
   Gln Asp Met Ile Ala Ala Leu Pro Glu Ala Thr His Glu Ala Ile Val
                   245                 250                 255
   Gly Val Gly Lys Gln Trp Ser Gly Ala Arg Ala Leu Glu Ala Leu Leu
               260                 265                 270
   Thr Val Ala Gly Glu Leu Arg Gly Pro Pro Leu Gln Leu Asp Thr Gly
           275                 280                 285
   Gln Leu Leu Lys Ile Ala Lys Arg Gly Gly Val Thr Ala Val Glu Ala
       290                 295                 300
   Val His Ala Trp Arg Asn Ala Leu Thr Gly Ala Pro Leu Asn Leu Thr
   305 310 315 320
   Pro Gln Gln Val Val Ala Ile Ala Ser Asn Gly Gly Gly Lys Gln Ala
   325 330 335
   Leu Glu Thr Val Gln Arg Leu Leu Pro Val Leu Cys Gln Ala His Gly
   340 345 350
   Leu Thr Pro Gln Gln Val Val Ala Ile Ala Ser Asn Gly Gly Gly Lys
   355 360 365
   Gln Ala Leu Glu Thr Val Gln Arg Leu Leu Pro Val Leu Cys Gln Ala
   370 375 380
   His Gly Leu Thr Pro Gln Gln Val Val Ala Ile Ala Ser Asn Gly Gly
   385 390 395 400
   Gly Lys Gln Ala Leu Glu Thr Val Gln Arg Leu Leu Pro Val Leu Cys
   405 410 415
   Gln Ala His Gly Leu Thr Pro Glu Gln Val Val Ala Ile Ala Ser His
   420 425 430
   Asp Gly Gly Lys Gln Ala Leu Glu Thr Val Gln Arg Leu Leu Pro Val
   435 440 445
   Leu Cys Gln Ala His Gly Leu Thr Pro Glu Gln Val Val Ala Ile Ala
   450 455 460
   Ser Asn Ile Gly Gly Lys Gln Ala Leu Glu Thr Val Gln Ala Leu Leu
   465 470 475 480
   Pro Val Leu Cys Gln Ala His Gly Leu Thr Pro Gln Gln Val Val Ala
   485 490 495
   Ile Ala Ser Asn Asn Gly Gly Lys Gln Ala Leu Glu Thr Val Gln Arg
   500 505 510
   Leu Leu Pro Val Leu Cys Gln Ala His Gly Leu Thr Pro Glu Gln Val
   515 520 525
   Val Ala Ile Ala Ser His Asp Gly Gly Lys Gln Ala Leu Glu Thr Val
   530 535 540
   Gln Arg Leu Leu Pro Val Leu Cys Gln Ala His Gly Leu Thr Pro Glu
   545                 550                 555                 560
   Gln Val Val Ala Ile Ala Ser Asn Ile Gly Gly Lys Gln Ala Leu Glu
                   565                 570                 575
   Thr Val Gln Ala Leu Leu Pro Val Leu Cys Gln Ala His Gly Leu Thr
               580                 585                 590
   Pro Glu Gln Val Val Ala Ile Ala Ser His Asp Gly Gly Lys Gln Ala
           595                 600                 605
   Leu Glu Thr Val Gln Arg Leu Leu Pro Val Leu Cys Gln Ala His Gly
       610                 615                 620
   Leu Thr Pro Glu Gln Val Val Ala Ile Ala Ser Asn Ile Gly Gly Lys
   625                 630                 635                 640
   Gln Ala Leu Glu Thr Val Gln Ala Leu Leu Pro Val Leu Cys Gln Ala
                   645                 650                 655
   His Gly Leu Thr Pro Glu Gln Val Val Ala Ile Ala Ser Asn Ile Gly
               660                 665                 670
   Gly Lys Gln Ala Leu Glu Thr Val Gln Ala Leu Leu Pro Val Leu Cys
           675                 680                 685
   Gln Ala His Gly Leu Thr Pro Glu Gln Val Val Ala Ile Ala Ser Asn
       690                 695                 700
   Ile Gly Gly Lys Gln Ala Leu Glu Thr Val Gln Ala Leu Leu Pro Val
   705                 710                 715                 720
   Leu Cys Gln Ala His Gly Leu Thr Pro Glu Gln Val Val Ala Ile Ala
                   725                 730                 735
   Ser Asn Ile Gly Gly Lys Gln Ala Leu Glu Thr Val Gln Ala Leu Leu
               740                 745                 750
   Pro Val Leu Cys Gln Ala His Gly Leu Thr Pro Gln Gln Val Val Ala
           755                 760                 765
   Ile Ala Ser Asn Asn Gly Gly Lys Gln Ala Leu Glu Thr Val Gln Arg
       770                 775                 780
   Leu Leu Pro Val Leu Cys Gln Ala His Gly Leu Thr Pro Glu Gln Val
   785                 790                 795                 800
   Val Ala Ile Ala Ser Asn Ile Gly Gly Lys Gln Ala Leu Glu Thr Val
                   805                 810                 815
   Gln Ala Leu Leu Pro Val Leu Cys Gln Ala His Gly Leu Thr Pro Gln
               820                 825                 830
   Gln Val Val Ala Ile Ala Ser Asn Gly Gly Gly Arg Pro Ala Leu Glu
           835                 840                 845
   Ser Ile Val Ala Gln Leu Ser Arg Pro Asp Pro Ala Leu Ala Ala Leu
       850                 855                 860
   Thr Asn Asp His Leu Val Ala Leu Ala Cys Leu Gly Gly Arg Pro Ala
   865                 870                 875                 880
   Leu Asp Ala Val Lys Lys Gly Leu Gly Asp Pro Ile Ser Arg Ser Gln
                   885                 890                 895
   Leu Val Lys Ser Glu Leu Glu Glu Lys Lys Ser Glu Leu Arg His Lys
               900                 905                 910
   Leu Lys Tyr Val Pro His Glu Tyr Ile Glu Leu Ile Glu Ile Ala Arg
           915                 920                 925
   Asn Ser Thr Gln Asp Arg Ile Leu Glu Met Lys Val Met Glu Phe Phe
       930                 935                 940
   Met Lys Val Tyr Gly Tyr Arg Gly Lys His Leu Gly Gly Ser Arg Lys
   945                 950                 955                 960
   Pro Asp Gly Ala Ile Tyr Thr Val Gly Ser Pro Ile Asp Tyr Gly Val
                   965                 970                 975
   Ile Val Asp Thr Lys Ala Tyr Ser Gly Gly Tyr Asn Leu Pro Ile Gly
               980                 985                 990
   Gln Ala Asp Glu Met Gln Arg Tyr  Val Glu Glu Asn Gln  Thr Arg Asn
           995                 1000                 1005
   Lys His  Ile Asn Pro Asn Glu  Trp Trp Lys Val Tyr  Pro Ser Ser
       1010                 1015                 1020
   Val Thr  Glu Phe Lys Phe Leu  Phe Val Ser Gly His  Phe Lys Gly
       1025                 1030                 1035
   Asn Tyr  Lys Ala Gln Leu Thr  Arg Leu Asn His Ile  Thr Asn Cys
       1040                 1045                 1050
   Asn Gly Ala Val Leu Ser Val Glu Glu Leu Leu Ile Gly Gly Glu
   1055 1060 1065
   Met Ile Lys Ala Gly Thr Leu Thr Leu Glu Glu Val Arg Arg Lys
   1070 1075 1080
   Phe Asn Asn Gly Glu Ile Asn Phe Ala Ala Ala
   1085 1090
<210> 7
   <211> 1088
   <212> PRT
   <213> artificial sequence
<220>
   <223> NR-TALEN-T02-L2
<400> 7
   Met Gly Asp Pro Lys Lys Lys Arg Lys Val Ile Asp Tyr Pro Tyr Asp
   1 5 10 15
   Val Pro Asp Tyr Ala Ile Asp Ile Ala Asp Pro Ile Arg Ser Arg Thr
   20 25 30
   Pro Ser Pro Ala Arg Glu Leu Leu Pro Gly Pro Gln Pro Asp Gly Val
   35 40 45
   Gln Pro Thr Ala Asp Arg Gly Val Ser Pro Pro Ala Gly Gly Pro Leu
   50 55 60
   Asp Gly Leu Pro Ala Arg Arg Thr Met Ser Arg Thr Arg Leu Pro Ser
   65 70 75 80
   Pro Pro Ala Pro Ser Pro Ala Phe Ser Ala Gly Ser Phe Ser Asp Leu
   85 90 95
   Leu Arg Gln Phe Asp Pro Ser Leu Phe Asn Thr Ser Leu Phe Asp Ser
   100 105 110
   Leu Pro Pro Phe Gly Ala His His Thr Glu Ala Ala Thr Gly Glu Trp
   115 120 125
   Asp Glu Val Gln Ser Gly Leu Arg Ala Ala Asp Ala Pro Pro Pro Thr
   130 135 140
   Met Arg Val Ala Val Thr Ala Ala Arg Pro Pro Arg Ala Lys Pro Ala
   145 150 155 160
   Pro Arg Arg Arg Ala Ala Gln Pro Ser Asp Ala Ser Pro Ala Ala Gln
                   165                 170                 175
   Val Asp Leu Arg Thr Leu Gly Tyr Ser Gln Gln Gln Gln Glu Lys Ile
               180                 185                 190
   Lys Pro Lys Val Arg Ser Thr Val Ala Gln His His Glu Ala Leu Val
           195                 200                 205
   Gly His Gly Phe Thr His Ala His Ile Val Ala Leu Ser Gln His Pro
       210                 215                 220
   Ala Ala Leu Gly Thr Val Ala Val Lys Tyr Gln Asp Met Ile Ala Ala
   225                 230                 235                 240
   Leu Pro Glu Ala Thr His Glu Ala Ile Val Gly Val Gly Lys Gln Trp
                   245                 250                 255
   Ser Gly Ala Arg Ala Leu Glu Ala Leu Leu Thr Val Ala Gly Glu Leu
               260                 265                 270
   Arg Gly Pro Pro Leu Gln Leu Asp Thr Gly Gln Leu Leu Lys Ile Ala
           275                 280                 285
   Lys Arg Gly Gly Val Thr Ala Val Glu Ala Val His Ala Trp Arg Asn
       290                 295                 300
   Ala Leu Thr Gly Ala Pro Leu Asn Leu Thr Pro Gln Gln Val Val Ala
   305                 310                 315                 320
   Ile Ala Ser Asn Asn Gly Gly Lys Gln Ala Leu Glu Thr Val Gln Arg
                   325                 330                 335
   Leu Leu Pro Val Leu Cys Gln Ala His Gly Leu Thr Pro Glu Gln Val
               340                 345                 350
   Val Ala Ile Ala Ser Asn Ile Gly Gly Lys Gln Ala Leu Glu Thr Val
           355                 360                 365
   Gln Ala Leu Leu Pro Val Leu Cys Gln Ala His Gly Leu Thr Pro Glu
       370                 375                 380
   Gln Val Val Ala Ile Ala Ser Asn Ile Gly Gly Lys Gln Ala Leu Glu
   385                 390                 395                 400
   Thr Val Gln Ala Leu Leu Pro Val Leu Cys Gln Ala His Gly Leu Thr
                   405                 410                 415
   Pro Gln Gln Val Val Ala Ile Ala Ser Asn Asn Gly Gly Lys Gln Ala
   420 425 430
   Leu Glu Thr Val Gln Arg Leu Leu Pro Val Leu Cys Gln Ala His Gly
   435 440 445
   Leu Thr Pro Glu Gln Val Val Ala Ile Ala Ser His Asp Gly Gly Lys
   450 455 460
   Gln Ala Leu Glu Thr Val Gln Arg Leu Leu Pro Val Leu Cys Gln Ala
   465 470 475 480
   His Gly Leu Thr Pro Glu Gln Val Val Ala Ile Ala Ser Asn Ile Gly
   485 490 495
   Gly Lys Gln Ala Leu Glu Thr Val Gln Ala Leu Leu Pro Val Leu Cys
   500 505 510
   Gln Ala His Gly Leu Thr Pro Gln Gln Val Val Ala Ile Ala Ser Asn
   515 520 525
   Asn Gly Gly Lys Gln Ala Leu Glu Thr Val Gln Arg Leu Leu Pro Val
   530 535 540
   Leu Cys Gln Ala His Gly Leu Thr Pro Glu Gln Val Val Ala Ile Ala
   545 550 555 560
   Ser His Asp Gly Gly Lys Gln Ala Leu Glu Thr Val Gln Arg Leu Leu
   565 570 575
   Pro Val Leu Cys Gln Ala His Gly Leu Thr Pro Glu Gln Val Val Ala
   580 585 590
   Ile Ala Ser Asn Ile Gly Gly Lys Gln Ala Leu Glu Thr Val Gln Ala
   595 600 605
   Leu Leu Pro Val Leu Cys Gln Ala His Gly Leu Thr Pro Gln Gln Val
   610 615 620
   Val Ala Ile Ala Ser Asn Gly Gly Gly Lys Gln Ala Leu Glu Thr Val
   625 630 635 640
   Gln Arg Leu Leu Pro Val Leu Cys Gln Ala His Gly Leu Thr Pro Glu
   645 650 655
   Gln Val Val Ala Ile Ala Ser His Asp Gly Gly Lys Gln Ala Leu Glu
   660 665 670
   Thr Val Gln Arg Leu Leu Pro Val Leu Cys Gln Ala His Gly Leu Thr
           675                 680                 685
   Pro Gln Gln Val Val Ala Ile Ala Ser Asn Asn Gly Gly Lys Gln Ala
       690                 695                 700
   Leu Glu Thr Val Gln Arg Leu Leu Pro Val Leu Cys Gln Ala His Gly
   705                 710                 715                 720
   Leu Thr Pro Glu Gln Val Val Ala Ile Ala Ser Asn Ile Gly Gly Lys
                   725                 730                 735
   Gln Ala Leu Glu Thr Val Gln Ala Leu Leu Pro Val Leu Cys Gln Ala
               740                 745                 750
   His Gly Leu Thr Pro Gln Gln Val Val Ala Ile Ala Ser Asn Gly Gly
           755                 760                 765
   Gly Lys Gln Ala Leu Glu Thr Val Gln Arg Leu Leu Pro Val Leu Cys
       770                 775                 780
   Gln Ala His Gly Leu Thr Pro Gln Gln Val Val Ala Ile Ala Ser Asn
   785                 790                 795                 800
   Gly Gly Gly Lys Gln Ala Leu Glu Thr Val Gln Arg Leu Leu Pro Val
                   805                 810                 815
   Leu Cys Gln Ala His Gly Leu Thr Pro Gln Gln Val Val Ala Ile Ala
               820                 825                 830
   Ser Asn Gly Gly Gly Arg Pro Ala Leu Glu Ser Ile Val Ala Gln Leu
           835                 840                 845
   Ser Arg Pro Asp Pro Ala Leu Ala Ala Leu Thr Asn Asp His Leu Val
       850                 855                 860
   Ala Leu Ala Cys Leu Gly Gly Arg Pro Ala Leu Asp Ala Val Lys Lys
   865                 870                 875                 880
   Gly Leu Gly Asp Pro Ile Ser Arg Ser Gln Leu Val Lys Ser Glu Leu
                   885                 890                 895
   Glu Glu Lys Lys Ser Glu Leu Arg His Lys Leu Lys Tyr Val Pro His
               900                 905                 910
   Glu Tyr Ile Glu Leu Ile Glu Ile Ala Arg Asn Ser Thr Gln Asp Arg
   915 920 925
   Ile Leu Glu Met Lys Val Met Glu Phe Phe Met Lys Val Tyr Gly Tyr
   930 935 940
   Arg Gly Lys His Leu Gly Gly Ser Arg Lys Pro Asp Gly Ala Ile Tyr
   945 950 955 960
   Thr Val Gly Ser Pro Ile Asp Tyr Gly Val Ile Val Asp Thr Lys Ala
   965 970 975
   Tyr Ser Gly Gly Tyr Asn Leu Pro Ile Gly Gln Ala Asp Glu Met Gln
   980 985 990
   Arg Tyr Val Glu Glu Asn Gln Thr Arg Asn Lys His Ile Asn Pro Asn
   995 1000 1005
   Glu Trp Trp Lys Val Tyr Pro Ser Ser Val Thr Glu Phe Lys Phe
   1010 1015 1020
   Leu Phe Val Ser Gly His Phe Lys Gly Asn Tyr Lys Ala Gln Leu
   1025 1030 1035
   Thr Arg Leu Asn His Ile Thr Asn Cys Asn Gly Ala Val Leu Ser
   1040 1045 1050
   Val Glu Glu Leu Leu Ile Gly Gly Glu Met Ile Lys Ala Gly Thr
   1055 1060 1065
   Leu Thr Leu Glu Glu Val Arg Arg Lys Phe Asn Asn Gly Glu Ile
   1070 1075 1080
   Asn Phe Ala Ala Asp
   1085
<210> 8
   <211> 1094
   <212> PRT
   <213> artificial sequence
<220>
   <223> NR-TALEN-T02-R2
<400> 8
   Met Gly Asp Pro Lys Lys Lys Arg Lys Val Ile Asp Lys Glu Thr Ala
   1               5                   10                  15
   Ala Ala Lys Phe Glu Arg Gln His Met Asp Ser Ile Asp Ile Ala Asp
               20                  25                  30
   Pro Ile Arg Ser Arg Thr Pro Ser Pro Ala Arg Glu Leu Leu Pro Gly
           35                  40                  45
   Pro Gln Pro Asp Gly Val Gln Pro Thr Ala Asp Arg Gly Val Ser Pro
       50                  55                  60
   Pro Ala Gly Gly Pro Leu Asp Gly Leu Pro Ala Arg Arg Thr Met Ser
   65                  70                  75                  80
   Arg Thr Arg Leu Pro Ser Pro Pro Ala Pro Ser Pro Ala Phe Ser Ala
                   85                  90                  95
   Gly Ser Phe Ser Asp Leu Leu Arg Gln Phe Asp Pro Ser Leu Phe Asn
               100                 105                 110
   Thr Ser Leu Phe Asp Ser Leu Pro Pro Phe Gly Ala His His Thr Glu
           115                 120                 125
   Ala Ala Thr Gly Glu Trp Asp Glu Val Gln Ser Gly Leu Arg Ala Ala
       130                 135                 140
   Asp Ala Pro Pro Pro Thr Met Arg Val Ala Val Thr Ala Ala Arg Pro
   145                 150                 155                 160
   Pro Arg Ala Lys Pro Ala Pro Arg Arg Arg Ala Ala Gln Pro Ser Asp
                   165                 170                 175
   Ala Ser Pro Ala Ala Gln Val Asp Leu Arg Thr Leu Gly Tyr Ser Gln
               180                 185                 190
   Gln Gln Gln Glu Lys Ile Lys Pro Lys Val Arg Ser Thr Val Ala Gln
           195                 200                 205
   His His Glu Ala Leu Val Gly His Gly Phe Thr His Ala His Ile Val
       210                 215                 220
   Ala Leu Ser Gln His Pro Ala Ala Leu Gly Thr Val Ala Val Lys Tyr
   225                 230                 235                 240
   Gln Asp Met Ile Ala Ala Leu Pro Glu Ala Thr His Glu Ala Ile Val
                   245                 250                 255
   Gly Val Gly Lys Gln Trp Ser Gly Ala Arg Ala Leu Glu Ala Leu Leu
               260                 265                 270
   Thr Val Ala Gly Glu Leu Arg Gly Pro Pro Leu Gln Leu Asp Thr Gly
               275                 280                 285
   Gln Leu Leu Lys Ile Ala Lys Arg Gly Gly Val Thr Ala Val Glu Ala
       290                 295                 300
   Val His Ala Trp Arg Asn Ala Leu Thr Gly Ala Pro Leu Asn Leu Thr
   305                 310                 315                 320
   Pro Gln Gln Val Val Ala Ile Ala Ser Asn Gly Gly Gly Lys Gln Ala
                   325                 330                 335
   Leu Glu Thr Val Gln Arg Leu Leu Pro Val Leu Cys Gln Ala His Gly
               340                 345                 350
   Leu Thr Pro Gln Gln Val Val Ala Ile Ala Ser Asn Asn Gly Gly Lys
           355                 360                 365
   Gln Ala Leu Glu Thr Val Gln Arg Leu Leu Pro Val Leu Cys Gln Ala
       370                 375                 380
   His Gly Leu Thr Pro Glu Gln Val Val Ala Ile Ala Ser His Asp Gly
   385                 390                 395                 400
   Gly Lys Gln Ala Leu Glu Thr Val Gln Arg Leu Leu Pro Val Leu Cys
                   405                 410                 415
   Gln Ala His Gly Leu Thr Pro Gln Gln Val Val Ala Ile Ala Ser Asn
               420                 425                 430
   Asn Gly Gly Lys Gln Ala Leu Glu Thr Val Gln Arg Leu Leu Pro Val
           435                 440                 445
   Leu Cys Gln Ala His Gly Leu Thr Pro Gln Gln Val Val Ala Ile Ala
       450                 455                 460
   Ser Asn Gly Gly Gly Lys Gln Ala Leu Glu Thr Val Gln Arg Leu Leu
   465                 470                 475                 480
   Pro Val Leu Cys Gln Ala His Gly Leu Thr Pro Glu Gln Val Val Ala
                   485                 490                 495
   Ile Ala Ser Asn Ile Gly Gly Lys Gln Ala Leu Glu Thr Val Gln Ala
               500                 505                 510
   Leu Leu Pro Val Leu Cys Gln Ala His Gly Leu Thr Pro Glu Gln Val
           515                 520                 525
   Val Ala Ile Ala Ser His Asp Gly Gly Lys Gln Ala Leu Glu Thr Val
   530 535 540
   Gln Arg Leu Leu Pro Val Leu Cys Gln Ala His Gly Leu Thr Pro Gln
   545 550 555 560
   Gln Val Val Ala Ile Ala Ser Asn Asn Gly Gly Lys Gln Ala Leu Glu
   565 570 575
   Thr Val Gln Arg Leu Leu Pro Val Leu Cys Gln Ala His Gly Leu Thr
   580 585 590
   Pro Gln Gln Val Val Ala Ile Ala Ser Asn Gly Gly Gly Lys Gln Ala
   595 600 605
   Leu Glu Thr Val Gln Arg Leu Leu Pro Val Leu Cys Gln Ala His Gly
   610 615 620
   Leu Thr Pro Glu Gln Val Val Ala Ile Ala Ser Asn Ile Gly Gly Lys
   625 630 635 640
   Gln Ala Leu Glu Thr Val Gln Ala Leu Leu Pro Val Leu Cys Gln Ala
   645 650 655
   His Gly Leu Thr Pro Glu Gln Val Val Ala Ile Ala Ser Asn Ile Gly
   660 665 670
   Gly Lys Gln Ala Leu Glu Thr Val Gln Ala Leu Leu Pro Val Leu Cys
   675 680 685
   Gln Ala His Gly Leu Thr Pro Gln Gln Val Val Ala Ile Ala Ser Asn
   690 695 700
   Gly Gly Gly Lys Gln Ala Leu Glu Thr Val Gln Arg Leu Leu Pro Val
   705 710 715 720
   Leu Cys Gln Ala His Gly Leu Thr Pro Gln Gln Val Val Ala Ile Ala
   725 730 735
   Ser Asn Asn Gly Gly Lys Gln Ala Leu Glu Thr Val Gln Arg Leu Leu
   740 745 750
   Pro Val Leu Cys Gln Ala His Gly Leu Thr Pro Glu Gln Val Val Ala
   755 760 765
   Ile Ala Ser His Asp Gly Gly Lys Gln Ala Leu Glu Thr Val Gln Arg
   770 775 780
   Leu Leu Pro Val Leu Cys Gln Ala His Gly Leu Thr Pro Glu Gln Val
   785 790 795 800
   Val Ala Ile Ala Ser Asn Ile Gly Gly Lys Gln Ala Leu Glu Thr Val
   805 810 815
   Gln Ala Leu Leu Pro Val Leu Cys Gln Ala His Gly Leu Thr Pro Gln
   820 825 830
   Gln Val Val Ala Ile Ala Ser Asn Gly Gly Gly Arg Pro Ala Leu Glu
   835 840 845
   Ser Ile Val Ala Gln Leu Ser Arg Pro Asp Pro Ala Leu Ala Ala Leu
   850 855 860
   Thr Asn Asp His Leu Val Ala Leu Ala Cys Leu Gly Gly Arg Pro Ala
   865 870 875 880
   Leu Asp Ala Val Lys Lys Gly Leu Gly Asp Pro Ile Ser Arg Ser Gln
   885 890 895
   Leu Val Lys Ser Glu Leu Glu Glu Lys Lys Ser Glu Leu Arg His Lys
   900 905 910
   Leu Lys Tyr Val Pro His Glu Tyr Ile Glu Leu Ile Glu Ile Ala Arg
   915 920 925
   Asn Ser Thr Gln Asp Arg Ile Leu Glu Met Lys Val Met Glu Phe Phe
   930 935 940
   Met Lys Val Tyr Gly Tyr Arg Gly Lys His Leu Gly Gly Ser Arg Lys
   945 950 955 960
   Pro Asp Gly Ala Ile Tyr Thr Val Gly Ser Pro Ile Asp Tyr Gly Val
   965 970 975
   Ile Val Asp Thr Lys Ala Tyr Ser Gly Gly Tyr Asn Leu Pro Ile Gly
   980 985 990
   Gln Ala Asp Glu Met Gln Arg Tyr Val Glu Glu Asn Gln Thr Arg Asn
   995 1000 1005
   Lys His Ile Asn Pro Asn Glu Trp Trp Lys Val Tyr Pro Ser Ser
   1010 1015 1020
   Val Thr Glu Phe Lys Phe Leu Phe Val Ser Gly His Phe Lys Gly
   1025 1030 1035
   Asn Tyr Lys Ala Gln Leu Thr Arg Leu Asn His Ile Thr Asn Cys
   1040 1045 1050
   Asn Gly Ala Val Leu Ser Val Glu Glu Leu Leu Ile Gly Gly Glu
   1055 1060 1065
   Met Ile Lys Ala Gly Thr Leu Thr Leu Glu Glu Val Arg Arg Lys
   1070 1075 1080
   Phe Asn Asn Gly Glu Ile Asn Phe Ala Ala Asp
   1085 1090
<210> 9
   <211> 3267
   <212> DNA
   <213> artificial sequence
<220>
   <223> UMPS-TALEN-T01-L1
<400> 9
<210> 10
   <211> 3285
   <212> DNA
   <213> artificial sequence
<220>
   <223> UMPS-TALEN-T01-R1
<400> 10
   atgggcgatc ctaaaaagaa acgtaaggtc atcgataagg agaccgccgc tgccaagttc     60
   gagagacagc acatggacag catcgatatc gccgacccca ttcgttcgcg cacaccaagt    120
   cctgcccgcg agcttctgcc cggaccccaa cccgatgggg ttcagccgac tgcagatcgt    180
   ggggtgtctc cgcctgccgg cggccccctg gatggcttgc cggctcggcg gacgatgtcc    240
   cggacccggc tgccatctcc ccctgccccc tcacctgcgt tctcggcggg cagcttcagt    300
   gacctgttac gtcagttcga tccgtcactt tttaatacat cgctttttga ttcattgcct    360
   cccttcggcg ctcaccatac agaggctgcc acaggcgagt gggatgaggt gcaatcgggt    420
   ctgcgggcag ccgacgcccc cccacccacc atgcgcgtgg ctgtcactgc cgcgcggccc    480
   ccgcgcgcca agccggcgcc gcgacgacgt gctgcgcaac cctccgacgc ttcgccggcg    540
   gcgcaggtgg atctacgcac gctcggctac agccagcagc aacaggagaa gatcaaaccg    600
   aaggttcgtt cgacagtggc gcagcaccac gaggcactgg tcggccacgg gtttacacac    660
   gcgcacatcg ttgcgttaag ccaacacccg gcagcgttag ggaccgtcgc tgtcaagtat    720
   caggacatga tcgcagcgtt gccagaggcg acacacgaag cgatcgttgg cgtcggcaaa    780
   cagtggtccg gcgcacgcgc tctggaggcc ttgctcacgg tggcgggaga gttgagaggt    840
   ccaccgttac agttggacac aggccaactt ctcaagattg caaaacgtgg cggcgtgacc    900
   gcagtggagg cagtgcatgc atggcgcaat gcactgacgg gtgccccgct caacttgacc    960
   ccccagcagg tggtggccat cgccagcaat ggcggtggca agcaggcgct ggagacggtc   1020
   cagcggctgt tgccggtgct gtgccaggcc cacggcttga ccccccagca ggtggtggcc   1080
   atcgccagca atggcggtgg caagcaggcg ctggagacgg tccagcggct gttgccggtg   1140
   ctgtgccagg cccacggctt gaccccccag caggtggtgg ccatcgccag caatggcggt   1200
   ggcaagcagg cgctggagac ggtccagcgg ctgttgccgg tgctgtgcca ggcccacggc   1260
   ttgaccccgg agcaggtggt ggccatcgcc agccacgatg gcggcaagca ggcgctggag 1320
   acggtccagc ggctgttgcc ggtgctgtgc caggcccacg gcttgacccc ggagcaggtg 1380
   gtggccatcg ccagcaatat tggtggcaag caggcgctgg agacggtgca ggcgctgttg 1440
   ccggtgctgt gccaggccca cggcttgacc ccccagcagg tggtggccat cgccagcaat 1500
   aatggtggca agcaggcgct ggagacggtc cagcggctgt tgccggtgct gtgccaggcc 1560
   cacggcttga ccccggagca ggtggtggcc atcgccagcc acgatggcgg caagcaggcg 1620
   ctggagacgg tccagcggct gttgccggtg ctgtgccagg cccacggctt gaccccggag 1680
   caggtggtgg ccatcgccag caatattggt ggcaagcagg cgctggagac ggtgcaggcg 1740
   ctgttgccgg tgctgtgcca ggcccacggc ttgaccccgg agcaggtggt ggccatcgcc 1800
   agccacgatg gcggcaagca ggcgctggag acggtccagc ggctgttgcc ggtgctgtgc 1860
   caggcccacg gcttgacccc ggagcaggtg gtggccatcg ccagcaatat tggtggcaag 1920
   caggcgctgg agacggtgca ggcgctgttg ccggtgctgt gccaggccca cggcttgacc 1980
   ccggagcagg tggtggccat cgccagcaat attggtggca agcaggcgct ggagacggtg 2040
   caggcgctgt tgccggtgct gtgccaggcc cacggcttga ccccggagca ggtggtggcc 2100
   atcgccagca atattggtgg caagcaggcg ctggagacgg tgcaggcgct gttgccggtg 2160
   ctgtgccagg cccacggctt gaccccggag caggtggtgg ccatcgccag caatattggt 2220
   ggcaagcagg cgctggagac ggtgcaggcg ctgttgccgg tgctgtgcca ggcccacggc 2280
   ttgacccccc agcaggtggt ggccatcgcc agcaataatg gtggcaagca ggcgctggag 2340
   acggtccagc ggctgttgcc ggtgctgtgc caggcccacg gcttgacccc ggagcaggtg 2400
   gtggccatcg ccagcaatat tggtggcaag caggcgctgg agacggtgca ggcgctgttg 2460
   ccggtgctgt gccaggccca cggcttgacc cctcagcagg tggtggccat cgccagcaat 2520
   ggcggcggca ggccggcgct ggagagcatt gttgcccagt tatctcgccc tgatccggcg 2580
   ttggccgcgt tgaccaacga ccacctcgtc gccttggcct gcctcggcgg gcgtcctgcg 2640
   ctggatgcag tgaaaaaggg attgggggat cctatcagcc gttcccagct ggtgaagtcc 2700
   gagctggagg agaagaaatc cgagttgagg cacaagctga agtacgtgcc ccacgagtac 2760
   atcgagctga tcgagatcgc ccggaacagc acccaggacc gtatcctgga gatgaaggtg 2820
   atggagttct tcatgaaggt gtacggctac aggggcaagc acctgggcgg ctccaggaag 2880
   cccgacggcg ccatctacac cgtgggctcc cccatcgact acggcgtgat cgtggacacc 2940
   aaggcctact ccggcggcta caacctgccc atcggccagg ccgacgaaat gcagaggtac 3000
   gtggaggaga accagaccag gaacaagcac atcaacccca acgagtggtg gaaggtgtac 3060
   ccctccagcg tgaccgagtt caagttcctg ttcgtgtccg gccacttcaa gggcaactac 3120
   aaggcccagc tgaccaggct gaaccacatc accaactgca acggcgccgt gctgtccgtg 3180
   gaggagctcc tgatcggcgg cgagatgatc aaggccggca ccctgaccct ggaggaggtg 3240
   aggaggaagt tcaacaacgg cgagatcaac ttcgcggccg cttga 3285
<210> 11
   <211> 3270
   <212> DNA
   <213> artificial sequence
<220>
   <223> NR-TALEN-T02-L2
<400> 11
   atgggcgatc ctaaaaagaa acgtaaggtc atcgattacc catacgatgt tccagattac      60
   gctatcgata tcgccgaccc cattcgttcg cgcacaccaa gtcctgcccg cgagcttctg     120
   cccggacccc aacccgatgg ggttcagccg actgcagatc gtggggtgtc tccgcctgcc     180
   ggcggccccc tggatggctt gccggctcgg cggacgatgt cccggacccg gctgccatct     240
   ccccctgccc cctcacctgc gttctcggcg ggcagcttca gtgacctgtt acgtcagttc    300
   gatccgtcac tttttaatac atcgcttttt gattcattgc ctcccttcgg cgctcaccat     360
   acagaggctg ccacaggcga gtgggatgag gtgcaatcgg gtctgcgggc agccgacgcc     420
   cccccaccca ccatgcgcgt ggctgtcact gccgcgcggc ccccgcgcgc caagccggcg     480
   ccgcgacgac gtgctgcgca accctccgac gcttcgccgg cggcgcaggt ggatctacgc     540
   acgctcggct acagccagca gcaacaggag aagatcaaac cgaaggttcg ttcgacagtg     600
   gcgcagcacc acgaggcact ggtcggccac gggtttacac acgcgcacat cgttgcgtta     660
   agccaacacc cggcagcgtt agggaccgtc gctgtcaagt atcaggacat gatcgcagcg     720
   ttgccagagg cgacacacga agcgatcgtt ggcgtcggca aacagtggtc cggcgcacgc     780
   gctctggagg ccttgctcac ggtggcggga gagttgagag gtccaccgtt acagttggac     840
   acaggccaac ttctcaagat tgcaaaacgt ggcggcgtga ccgcagtgga ggcagtgcat     900
   gcatggcgca atgcactgac gggtgccccg ctcaacttga ccccccagca ggtggtggcc     960
   atcgccagca ataatggtgg caagcaggcg ctggagacgg tccagcggct gttgccggtg    1020
   ctgtgccagg cccacggctt gaccccggag caggtggtgg ccatcgccag caatattggt    1080
   ggcaagcagg cgctggagac ggtgcaggcg ctgttgccgg tgctgtgcca ggcccacggc    1140
   ttgaccccgg agcaggtggt ggccatcgcc agcaatattg gtggcaagca ggcgctggag    1200
   acggtgcagg cgctgttgcc ggtgctgtgc caggcccacg gcttgacccc ccagcaggtg    1260
   gtggccatcg ccagcaataa tggtggcaag caggcgctgg agacggtcca gcggctgttg    1320
   ccggtgctgt gccaggccca cggcttgacc ccggagcagg tggtggccat cgccagccac    1380
   gatggcggca agcaggcgct ggagacggtc cagcggctgt tgccggtgct gtgccaggcc    1440
   cacggcttga ccccggagca ggtggtggcc atcgccagca atattggtgg caagcaggcg 1500
   ctggagacgg tgcaggcgct gttgccggtg ctgtgccagg cccacggctt gaccccccag 1560
   caggtggtgg ccatcgccag caataatggt ggcaagcagg cgctggagac ggtccagcgg 1620
   ctgttgccgg tgctgtgcca ggcccacggc ttgaccccgg agcaggtggt ggccatcgcc 1680
   agccacgatg gcggcaagca ggcgctggag acggtccagc ggctgttgcc ggtgctgtgc 1740
   caggcccacg gcttgacccc ggagcaggtg gtggccatcg ccagcaatat tggtggcaag 1800
   caggcgctgg agacggtgca ggcgctgttg ccggtgctgt gccaggccca cggcttgacc 1860
   ccccagcagg tggtggccat cgccagcaat ggcggtggca agcaggcgct ggagacggtc 1920
   cagcggctgt tgccggtgct gtgccaggcc cacggcttga ccccggagca ggtggtggcc 1980
   atcgccagcc acgatggcgg caagcaggcg ctggagacgg tccagcggct gttgccggtg 2040
   ctgtgccagg cccacggctt gaccccccag caggtggtgg ccatcgccag caataatggt 2100
   ggcaagcagg cgctggagac ggtccagcgg ctgttgccgg tgctgtgcca ggcccacggc 2160
   ttgaccccgg agcaggtggt ggccatcgcc agcaatattg gtggcaagca ggcgctggag 2220
   acggtgcagg cgctgttgcc ggtgctgtgc caggcccacg gcttgacccc ccagcaggtg 2280
   gtggccatcg ccagcaatgg cggtggcaag caggcgctgg agacggtcca gcggctgttg 2340
   ccggtgctgt gccaggccca cggcttgacc ccccagcagg tggtggccat cgccagcaat 2400
   ggcggtggca agcaggcgct ggagacggtc cagcggctgt tgccggtgct gtgccaggcc 2460
   cacggcttga cccctcagca ggtggtggcc atcgccagca atggcggcgg caggccggcg 2520
   ctggagagca ttgttgccca gttatctcgc cctgatccgg cgttggccgc gttgaccaac 2580
   gaccacctcg tcgccttggc ctgcctcggc gggcgtcctg cgctggatgc agtgaaaaag 2640
   ggattggggg atcctatcag ccgttcccag ctggtgaagt ccgagctgga ggagaagaaa 2700
   tccgagttga ggcacaagct gaagtacgtg ccccacgagt acatcgagct gatcgagatc 2760
   gcccggaaca gcacccagga ccgtatcctg gagatgaagg tgatggagtt cttcatgaag 2820
   gtgtacggct acaggggcaa gcacctgggc ggctccagga agcccgacgg cgccatctac 2880
   accgtgggct cccccatcga ctacggcgtg atcgtggaca ccaaggccta ctccggcggc 2940
   tacaacctgc ccatcggcca ggccgacgaa atgcagaggt acgtggagga gaaccagacc 3000
   aggaacaagc acatcaaccc caacgagtgg tggaaggtgt acccctccag cgtgaccgag 3060
   ttcaagttcc tgttcgtgtc cggccacttc aagggcaact acaaggccca gctgaccagg 3120
   ctgaaccaca tcaccaactg caacggcgcc gtgctgtccg tggaggagct cctgatcggc 3180
   ggcgagatga tcaaggccgg caccctgacc ctggaggagg tgaggaggaa gttcaacaac 3240
   ggcgagatca acttcgcggc cgactgataa 3270
<210> 12
   <211> 3288
   <212> DNA
   <213> artificial sequence
<220>
   <223> NR-TALEN-T02-R2
<400> 12
   atgggcgatc ctaaaaagaa acgtaaggtc atcgataagg agaccgccgc tgccaagttc      60
   gagagacagc acatggacag catcgatatc gccgacccca ttcgttcgcg cacaccaagt     120
   cctgcccgcg agcttctgcc cggaccccaa cccgatgggg ttcagccgac tgcagatcgt     180
   ggggtgtctc cgcctgccgg cggccccctg gatggcttgc cggctcggcg gacgatgtcc     240
   cggacccggc tgccatctcc ccctgccccc tcacctgcgt tctcggcggg cagcttcagt     300
   gacctgttac gtcagttcga tccgtcactt tttaatacat cgctttttga ttcattgcct     360
   cccttcggcg ctcaccatac agaggctgcc acaggcgagt gggatgaggt gcaatcgggt     420
   ctgcgggcag ccgacgcccc cccacccacc atgcgcgtgg ctgtcactgc cgcgcggccc     480
   ccgcgcgcca agccggcgcc gcgacgacgt gctgcgcaac cctccgacgc ttcgccggcg     540
   gcgcaggtgg atctacgcac gctcggctac agccagcagc aacaggagaa gatcaaaccg     600
   aaggttcgtt cgacagtggc gcagcaccac gaggcactgg tcggccacgg gtttacacac     660
   gcgcacatcg ttgcgttaag ccaacacccg gcagcgttag ggaccgtcgc tgtcaagtat     720
   caggacatga tcgcagcgtt gccagaggcg acacacgaag cgatcgttgg cgtcggcaaa     780
   cagtggtccg gcgcacgcgc tctggaggcc ttgctcacgg tggcgggaga gttgagaggt     840
   ccaccgttac agttggacac aggccaactt ctcaagattg caaaacgtgg cggcgtgacc     900
   gcagtggagg cagtgcatgc atggcgcaat gcactgacgg gtgccccgct caacttgacc     960
   ccccagcagg tggtggccat cgccagcaat ggcggtggca agcaggcgct ggagacggtc    1020
   cagcggctgt tgccggtgct gtgccaggcc cacggcttga ccccccagca ggtggtggcc    1080
   atcgccagca ataatggtgg caagcaggcg ctggagacgg tccagcggct gttgccggtg    1140
   ctgtgccagg cccacggctt gaccccggag caggtggtgg ccatcgccag ccacgatggc    1200
   ggcaagcagg cgctggagac ggtccagcgg ctgttgccgg tgctgtgcca ggcccacggc    1260
   ttgacccccc agcaggtggt ggccatcgcc agcaataatg gtggcaagca ggcgctggag    1320
   acggtccagc ggctgttgcc ggtgctgtgc caggcccacg gcttgacccc ccagcaggtg    1380
   gtggccatcg ccagcaatgg cggtggcaag caggcgctgg agacggtcca gcggctgttg    1440
   ccggtgctgt gccaggccca cggcttgacc ccggagcagg tggtggccat cgccagcaat    1500
   attggtggca agcaggcgct ggagacggtg caggcgctgt tgccggtgct gtgccaggcc    1560
   cacggcttga ccccggagca ggtggtggcc atcgccagcc acgatggcgg caagcaggcg    1620
   ctggagacgg tccagcggct gttgccggtg ctgtgccagg cccacggctt gaccccccag 1680
   caggtggtgg ccatcgccag caataatggt ggcaagcagg cgctggagac ggtccagcgg 1740
   ctgttgccgg tgctgtgcca ggcccacggc ttgacccccc agcaggtggt ggccatcgcc 1800
   agcaatggcg gtggcaagca ggcgctggag acggtccagc ggctgttgcc ggtgctgtgc 1860
   caggcccacg gcttgacccc ggagcaggtg gtggccatcg ccagcaatat tggtggcaag 1920
   caggcgctgg agacggtgca ggcgctgttg ccggtgctgt gccaggccca cggcttgacc 1980
   ccggagcagg tggtggccat cgccagcaat attggtggca agcaggcgct ggagacggtg 2040
   caggcgctgt tgccggtgct gtgccaggcc cacggcttga ccccccagca ggtggtggcc 2100
   atcgccagca atggcggtgg caagcaggcg ctggagacgg tccagcggct gttgccggtg 2160
   ctgtgccagg cccacggctt gaccccccag caggtggtgg ccatcgccag caataatggt 2220
   ggcaagcagg cgctggagac ggtccagcgg ctgttgccgg tgctgtgcca ggcccacggc 2280
   ttgaccccgg agcaggtggt ggccatcgcc agccacgatg gcggcaagca ggcgctggag 2340
   acggtccagc ggctgttgcc ggtgctgtgc caggcccacg gcttgacccc ggagcaggtg 2400
   gtggccatcg ccagcaatat tggtggcaag caggcgctgg agacggtgca ggcgctgttg 2460
   ccggtgctgt gccaggccca cggcttgacc cctcagcagg tggtggccat cgccagcaat 2520
   ggcggcggca ggccggcgct ggagagcatt gttgcccagt tatctcgccc tgatccggcg 2580
   ttggccgcgt tgaccaacga ccacctcgtc gccttggcct gcctcggcgg gcgtcctgcg 2640
   ctggatgcag tgaaaaaggg attgggggat cctatcagcc gttcccagct ggtgaagtcc 2700
   gagctggagg agaagaaatc cgagttgagg cacaagctga agtacgtgcc ccacgagtac 2760
   atcgagctga tcgagatcgc ccggaacagc acccaggacc gtatcctgga gatgaaggtg 2820
   atggagttct tcatgaaggt gtacggctac aggggcaagc acctgggcgg ctccaggaag 2880
   cccgacggcg ccatctacac cgtgggctcc cccatcgact acggcgtgat cgtggacacc 2940
   aaggcctact ccggcggcta caacctgccc atcggccagg ccgacgaaat gcagaggtac 3000
   gtggaggaga accagaccag gaacaagcac atcaacccca acgagtggtg gaaggtgtac 3060
   ccctccagcg tgaccgagtt caagttcctg ttcgtgtccg gccacttcaa gggcaactac 3120
   aaggcccagc tgaccaggct gaaccacatc accaactgca acggcgccgt gctgtccgtg 3180
   gaggagctcc tgatcggcgg cgagatgatc aaggccggca ccctgaccct ggaggaggtg 3240
   aggaggaagt tcaacaacgg cgagatcaac ttcgcggccg actgataa 3288
<210> 13
   <211> 5922
   <212> DNA
   <213> artificial sequence
<220>
   <223> plasmid vector: pCLS20603
<400> 13
   gggtacgttt aaacgtatta attaagacct agcatgtgag caaaaggcca gcaaaaggcc      60
   aggaaccgta aaaaggccgc gttgctggcg tttttccata ggctccgccc ccctgacgag     120
   catcacaaaa atcgacgctc aagtcagagg tggcgaaacc cgacaggact ataaagatac     180
   caggcgtttc cccctggaag ctccctcgtg cgctctcctg ttccgaccct gccgcttacc     240
   ggatacctgt ccgcctttct cccttcggga agcgtggcgc tttctcatag ctcacgctgt     300
   aggtatctca gttcggtgta ggtcgttcgc tccaagctgg gctgtgtgca cgaacccccc     360
   gttcagcccg accgctgcgc cttatccggt aactatcgtc ttgagtccaa cccggtaaga     420
   cacgacttat cgccactggc agcagccact ggtaacagga ttagcagagc gaggtatgta     480
   ggcggtgcta cagagttctt gaagtggtgg cctaactacg gctacactag aaggacagta     540
   tttggtatct gcgctctgct gaagccagtt accttcggaa aaagagttgg tagctcttga     600
   tccggcaaac aaaccaccgc tggtagcggt ggtttttttg tttgcaagca gcagattacg     660
   cgcagaaaaa aaggatctca agaagatcct ttgatctttt ctacggggtc tgacgctcag     720
   tggaacgaaa actcacgtta agggattttg gtcatgagat tatcaaaaag gatcttcacc     780
   tagatccttt taaattaaaa atgaagtttt aaatcaatct aaagtatata tgagtaaact     840
   tggtctgaca gttaccaatg cttaatcagt gaggcaccta tctcagcgat ctgtctattt     900
   cgttcatcca tagttgcctg actccccgtc gtgtagataa ctacgatacg ggagggctta     960
   ccatctggcc ccagtgctgc aatgataccg cgagacccac gctcaccggc tccagattta    1020
   tcagcaataa accagccagc cggaagggcc gagcgcagaa gtggtcctgc aactttatcc    1080
   gcctccatcc agtctattaa ttgttgccgg gaagctagag taagtagttc gccagttaat    1140
   agtttgcgca acgttgttgc cattgctaca ggcatcgtgg tgtcacgctc gtcgtttggt    1200
   atggcttcat tcagctccgg ttcccaacga tcaaggcgag ttacatgatc ccccatgttg    1260
   tgcaaaaaag cggttagctc cttcggtcct ccgatcgttg tcagaagtaa gttggccgca    1320
   gtgttatcac tcatggttat ggcagcactg cataattctc ttactgtcat gccatccgta    1380
   agatgctttt ctgtgactgg tgagtactca accaagtcat tctgagaata gtgtatgcgg    1440
   cgaccgagtt gctcttgccc ggcgtcaata cgggataata ccgcgccaca tagcagaact    1500
   ttaaaagtgc tcatcattgg aaaacgttct tcggggcgaa aactctcaag gatcttaccg    1560
   ctgttgagat ccagttcgat gtaacccact cgtgcaccca actgatcttc agcatctttt    1620
   actttcacca gcgtttctgg gtgagcaaaa acaggaaggc aaaatgccgc aaaaaaggga    1680
   ataagggcga cacggaaatg ttgaatactc atactcttcc tttttcaata ttattgaagc    1740
   atttatcagg gttattgtct catgagcgga tacatatttg aatgtattta gaaaaataaa    1800
   caaatagggg ttccgcgcac atttccccga aaagtgccac ctgacaaact tggtaccata 1860
   actagttcgg cgcgccaatc tcgcctattc atggtgtata aaagttcaac atccaaagct 1920
   agaacttttg gaaagagaaa gaatgtccga atagggcacg gcgtgccgta ttgttggagt 1980
   ggactagcag aaagtgagga aggcacagga tgagtttcct cgagacacat agcttcagcg 2040
   tcgtgtaggc taggcagagg tgagttttct cgagacatac cttcagcgtc gtcttcactg 2100
   tcacagtcaa ctgacagtaa tcgttgatcc ggagagattc aaaattcaat ctgtttggac 2160
   ctggataaga cacaagagcg acatcctgac atgaacgccg taaacagcaa atcctggttg 2220
   aacacgtatc cttttggggg cctccagcta cgacgctcgc cccagctggg gcttccttac 2280
   tatacacagc gcatatttca cggttgccag aaccatgggc gatcctaaaa agaaacgtaa 2340
   ggtcatcgat tacccatacg atgttccaga ttacgctatc gatatcgccg accccattcg 2400
   ttcgcgcaca ccaagtcctg cccgcgagct tctgcccgga ccccaacccg atggggttca 2460
   gccgactgca gatcgtgggg tgtctccgcc tgccggcggc cccctggatg gcttgccggc 2520
   tcggcggacg atgtcccgga cccggctgcc atctccccct gccccctcac ctgcgttctc 2580
   ggcgggcagc ttcagtgacc tgttacgtca gttcgatccg tcacttttta atacatcgct 2640
   ttttgattca ttgcctccct tcggcgctca ccatacagag gctgccacag gcgagtggga 2700
   tgaggtgcaa tcgggtctgc gggcagccga cgccccccca cccaccatgc gcgtggctgt 2760
   cactgccgcg cggcccccgc gcgccaagcc ggcgccgcga cgacgtgctg cgcaaccctc 2820
   cgacgcttcg ccggcggcgc aggtggatct acgcacgctc ggctacagcc agcagcaaca 2880
   ggagaagatc aaaccgaagg ttcgttcgac agtggcgcag caccacgagg cactggtcgg 2940
   ccacgggttt acacacgcgc acatcgttgc gttaagccaa cacccggcag cgttagggac 3000
   cgtcgctgtc aagtatcagg acatgatcgc agcgttgcca gaggcgacac acgaagcgat 3060
   cgttggcgtc ggcaaacagt ggtccggcgc acgcgctctg gaggccttgc tcacggtggc 3120
   gggagagttg agaggtccac cgttacagtt ggacacaggc caacttctca agattgcaaa 3180
   acgtggcggc gtgaccgcag tggaggcagt gcatgcatgg cgcaatgcac tgacgggtgc 3240
   cccgctcaac ttgacccccc agcaggtggt ggccatcgcc agcaatggcg gtggcaagca 3300
   ggcgctggag acggtccagc ggctgttgcc ggtgctgtgc caggcccacg gcttgacccc 3360
   ccagcaggtg gtggccatcg ccagcaatgg cggtggcaag caggcgctgg agacggtcca 3420
   gcggctgttg ccggtgctgt gccaggccca cggcttgacc ccggagcagg tggtggccat 3480
   cgccagcaat attggtggca agcaggcgct ggagacggtg caggcgctgt tgccggtgct 3540
   gtgccaggcc cacggcttga ccccccagca ggtggtggcc atcgccagca ataatggtgg 3600
   caagcaggcg ctggagacgg tccagcggct gttgccggtg ctgtgccagg cccacggctt 3660
   gaccccccag caggtggtgg ccatcgccag caatggcggt ggcaagcagg cgctggagac 3720
   ggtccagcgg ctgttgccgg tgctgtgcca ggcccacggc ttgaccccgg agcaggtggt 3780
   ggccatcgcc agccacgatg gcggcaagca ggcgctggag acggtccagc ggctgttgcc 3840
   ggtgctgtgc caggcccacg gcttgacccc ccagcaggtg gtggccatcg ccagcaatgg 3900
   cggtggcaag caggcgctgg agacggtcca gcggctgttg ccggtgctgt gccaggccca 3960
   cggcttgacc ccccagcagg tggtggccat cgccagcaat aatggtggca agcaggcgct 4020
   ggagacggtc cagcggctgt tgccggtgct gtgccaggcc cacggcttga ccccccagca 4080
   ggtggtggcc atcgccagca atggcggtgg caagcaggcg ctggagacgg tccagcggct 4140
   gttgccggtg ctgtgccagg cccacggctt gaccccggag caggtggtgg ccatcgccag 4200
   ccacgatggc ggcaagcagg cgctggagac ggtccagcgg ctgttgccgg tgctgtgcca 4260
   ggcccacggc ttgacccccc agcaggtggt ggccatcgcc agcaatggcg gtggcaagca 4320
   ggcgctggag acggtccagc ggctgttgcc ggtgctgtgc caggcccacg gcttgacccc 4380
   ggagcaggtg gtggccatcg ccagccacga tggcggcaag caggcgctgg agacggtcca 4440
   gcggctgttg ccggtgctgt gccaggccca cggcttgacc ccccagcagg tggtggccat 4500
   cgccagcaat ggcggtggca agcaggcgct ggagacggtc cagcggctgt tgccggtgct 4560
   gtgccaggcc cacggcttga ccccggagca ggtggtggcc atcgccagca atattggtgg 4620
   caagcaggcg ctggagacgg tgcaggcgct gttgccggtg ctgtgccagg cccacggctt 4680
   gaccccccag caggtggtgg ccatcgccag caataatggt ggcaagcagg cgctggagac 4740
   ggtccagcgg ctgttgccgg tgctgtgcca ggcccacggc ttgacccctc agcaggtggt 4800
   ggccatcgcc agcaatggcg gcggcaggcc ggcgctggag agcattgttg cccagttatc 4860
   tcgccctgat ccggcgttgg ccgcgttgac caacgaccac ctcgtcgcct tggcctgcct 4920
   cggcgggcgt cctgcgctgg atgcagtgaa aaagggattg gggatcctat tcagccgttc 4980
   ccagctggtg aagtccgagc tggaggagaa gaaatccgag ttgaggcaca agctgaagta 5040
   cgtgccccac gagtacatcg agctgatcga gatcgcccgg aacagcaccc aggaccgtat 5100
   cctggagatg aaggtgatgg agttcttcat gaaggtgtac ggctacaggg gcaagcacct 5160
   gggcggctcc aggaagcccg acggcgccat ctacaccgtg ggctccccca tcgactacgg 5220
   cgtgatcgtg gacaccaagg cctactccgg cggctacaac ctgcccatcg gccaggccga 5280
   cgaaatgcag aggtacgtgg aggagaacca gaccaggaac aagcacatca accccaacga 5340
   gtggtggaag gtgtacccct ccagcgtgac cgagttcaag ttcctgttcg tgtccggcca 5400
   cttcaagggc aactacaagg cccagctgac caggctgaac cacatcacca actgcaacgg 5460
   cgccgtgctg tccgtggagg agctcctgat cggcggcgag atgatcaagg ccggcaccct 5520
   gaccctggag gaggtgagga ggaagttcaa caacggcgag atcaacttcg cggccgcttg 5580
   ataactcgag cgatcctcta gacgagctcc tcgagcctgc agcagctgaa gctttaagat     5640
   ccaatggcaa ggaccaagtg ctggaacttg ttttgcttta gcagatctag atcgagctac     5700
   ctcgactttg gctgggacac tttcagtgag gacaagaagc ttcagaagcg tgctatcgaa     5760
   ctcaaccagg gacgtgcggc acaaatgggc atccttgctc tcatggtgca cgaacagttg     5820
   ggagtctcta tccttcctta aaaatttaat tttcattagt tgcagtcact ccgctttggt     5880
   ttcacagtca ggaataacac tagctcgtct tcatatcctg ca                        5922
<210> 14
   <211> 5940
   <212> DNA
   <213> artificial sequence
<220>
   <223> plasmid vector: pCLS20604
<400> 14
   gggtacgttt aaacgtatta attaagacct agcatgtgag caaaaggcca gcaaaaggcc 60
   aggaaccgta aaaaggccgc gttgctggcg tttttccata ggctccgccc ccctgacgag 120
   catcacaaaa atcgacgctc aagtcagagg tggcgaaacc cgacaggact ataaagatac 180
   caggcgtttc cccctggaag ctccctcgtg cgctctcctg ttccgaccct gccgcttacc 240
   ggatacctgt ccgcctttct cccttcggga agcgtggcgc tttctcatag ctcacgctgt 300
   aggtatctca gttcggtgta ggtcgttcgc tccaagctgg gctgtgtgca cgaacccccc 360
   gttcagcccg accgctgcgc cttatccggt aactatcgtc ttgagtccaa cccggtaaga 420
   cacgacttat cgccactggc agcagccact ggtaacagga ttagcagagc gaggtatgta 480
   ggcggtgcta cagagttctt gaagtggtgg cctaactacg gctacactag aaggacagta 540
   tttggtatct gcgctctgct gaagccagtt accttcggaa aaagagttgg tagctcttga 600
   tccggcaaac aaaccaccgc tggtagcggt ggtttttttg tttgcaagca gcagattacg 660
   cgcagaaaaa aaggatctca agaagatcct ttgatctttt ctacggggtc tgacgctcag 720
   tggaacgaaa actcacgtta agggattttg gtcatgagat tatcaaaaag gatcttcacc 780
   tagatccttt taaattaaaa atgaagtttt aaatcaatct aaagtatata tgagtaaact 840
   tggtctgaca gttaccaatg cttaatcagt gaggcaccta tctcagcgat ctgtctattt 900
   cgttcatcca tagttgcctg actccccgtc gtgtagataa ctacgatacg ggagggctta 960
   ccatctggcc ccagtgctgc aatgataccg cgagacccac gctcaccggc tccagattta 1020
   tcagcaataa accagccagc cggaagggcc gagcgcagaa gtggtcctgc aactttatcc 1080
   gcctccatcc agtctattaa ttgttgccgg gaagctagag taagtagttc gccagttaat 1140
   agtttgcgca acgttgttgc cattgctaca ggcatcgtgg tgtcacgctc gtcgtttggt 1200
   atggcttcat tcagctccgg ttcccaacga tcaaggcgag ttacatgatc ccccatgttg 1260
   tgcaaaaaag cggttagctc cttcggtcct ccgatcgttg tcagaagtaa gttggccgca 1320
   gtgttatcac tcatggttat ggcagcactg cataattctc ttactgtcat gccatccgta 1380
   agatgctttt ctgtgactgg tgagtactca accaagtcat tctgagaata gtgtatgcgg 1440
   cgaccgagtt gctcttgccc ggcgtcaata cgggataata ccgcgccaca tagcagaact 1500
   ttaaaagtgc tcatcattgg aaaacgttct tcggggcgaa aactctcaag gatcttaccg 1560
   ctgttgagat ccagttcgat gtaacccact cgtgcaccca actgatcttc agcatctttt 1620
   actttcacca gcgtttctgg gtgagcaaaa acaggaaggc aaaatgccgc aaaaaaggga 1680
   ataagggcga cacggaaatg ttgaatactc atactcttcc tttttcaata ttattgaagc 1740
   atttatcagg gttattgtct catgagcgga tacatatttg aatgtattta gaaaaataaa 1800
   caaatagggg ttccgcgcac atttccccga aaagtgccac ctgacaaact tggtaccata 1860
   actagttcgg cgcgccaatc tcgcctattc atggtgtata aaagttcaac atccaaagct 1920
   agaacttttg gaaagagaaa gaatgtccga atagggcacg gcgtgccgta ttgttggagt 1980
   ggactagcag aaagtgagga aggcacagga tgagtttcct cgagacacat agcttcagcg 2040
   tcgtgtaggc taggcagagg tgagttttct cgagacatac cttcagcgtc gtcttcactg 2100
   tcacagtcaa ctgacagtaa tcgttgatcc ggagagattc aaaattcaat ctgtttggac 2160
   ctggataaga cacaagagcg acatcctgac atgaacgccg taaacagcaa atcctggttg 2220
   aacacgtatc cttttggggg cctccagcta cgacgctcgc cccagctggg gcttccttac 2280
   tatacacagc gcatatttca cggttgccag aaccatgggc gatcctaaaa agaaacgtaa 2340
   ggtcatcgat aaggagaccg ccgctgccaa gttcgagaga cagcacatgg acagcatcga 2400
   tatcgccgac cccattcgtt cgcgcacacc aagtcctgcc cgcgagcttc tgcccggacc 2460
   ccaacccgat ggggttcagc cgactgcaga tcgtggggtg tctccgcctg ccggcggccc 2520
   cctggatggc ttgccggctc ggcggacgat gtcccggacc cggctgccat ctccccctgc 2580
   cccctcacct gcgttctcgg cgggcagctt cagtgacctg ttacgtcagt tcgatccgtc 2640
   actttttaat acatcgcttt ttgattcatt gcctcccttc ggcgctcacc atacagaggc 2700
   tgccacaggc gagtgggatg aggtgcaatc gggtctgcgg gcagccgacg cccccccacc 2760
   caccatgcgc gtggctgtca ctgccgcgcg gcccccgcgc gccaagccgg cgccgcgacg 2820
   acgtgctgcg caaccctccg acgcttcgcc ggcggcgcag gtggatctac gcacgctcgg 2880
   ctacagccag cagcaacagg agaagatcaa accgaaggtt cgttcgacag tggcgcagca 2940
   ccacgaggca ctggtcggcc acgggtttac acacgcgcac atcgttgcgt taagccaaca 3000
   cccggcagcg ttagggaccg tcgctgtcaa gtatcaggac atgatcgcag cgttgccaga 3060
   ggcgacacac gaagcgatcg ttggcgtcgg caaacagtgg tccggcgcac gcgctctgga 3120
   ggccttgctc acggtggcgg gagagttgag aggtccaccg ttacagttgg acacaggcca 3180
   acttctcaag attgcaaaac gtggcggcgt gaccgcagtg gaggcagtgc atgcatggcg 3240
   caatgcactg acgggtgccc cgctcaactt gaccccccag caggtggtgg ccatcgccag 3300
   caatggcggt ggcaagcagg cgctggagac ggtccagcgg ctgttgccgg tgctgtgcca 3360
   ggcccacggc ttgacccccc agcaggtggt ggccatcgcc agcaatggcg gtggcaagca 3420
   ggcgctggag acggtccagc ggctgttgcc ggtgctgtgc caggcccacg gcttgacccc 3480
   ccagcaggtg gtggccatcg ccagcaatgg cggtggcaag caggcgctgg agacggtcca 3540
   gcggctgttg ccggtgctgt gccaggccca cggcttgacc ccggagcagg tggtggccat 3600
   cgccagccac gatggcggca agcaggcgct ggagacggtc cagcggctgt tgccggtgct 3660
   gtgccaggcc cacggcttga ccccggagca ggtggtggcc atcgccagca atattggtgg 3720
   caagcaggcg ctggagacgg tgcaggcgct gttgccggtg ctgtgccagg cccacggctt 3780
   gaccccccag caggtggtgg ccatcgccag caataatggt ggcaagcagg cgctggagac 3840
   ggtccagcgg ctgttgccgg tgctgtgcca ggcccacggc ttgaccccgg agcaggtggt 3900
   ggccatcgcc agccacgatg gcggcaagca ggcgctggag acggtccagc ggctgttgcc 3960
   ggtgctgtgc caggcccacg gcttgacccc ggagcaggtg gtggccatcg ccagcaatat 4020
   tggtggcaag caggcgctgg agacggtgca ggcgctgttg ccggtgctgt gccaggccca 4080
   cggcttgacc ccggagcagg tggtggccat cgccagccac gatggcggca agcaggcgct 4140
   ggagacggtc cagcggctgt tgccggtgct gtgccaggcc cacggcttga ccccggagca 4200
   ggtggtggcc atcgccagca atattggtgg caagcaggcg ctggagacgg tgcaggcgct 4260
   gttgccggtg ctgtgccagg cccacggctt gaccccggag caggtggtgg ccatcgccag 4320
   caatattggt ggcaagcagg cgctggagac ggtgcaggcg ctgttgccgg tgctgtgcca 4380
   ggcccacggc ttgaccccgg agcaggtggt ggccatcgcc agcaatattg gtggcaagca 4440
   ggcgctggag acggtgcagg cgctgttgcc ggtgctgtgc caggcccacg gcttgacccc 4500
   ggagcaggtg gtggccatcg ccagcaatat tggtggcaag caggcgctgg agacggtgca 4560
   ggcgctgttg ccggtgctgt gccaggccca cggcttgacc ccccagcagg tggtggccat 4620
   cgccagcaat aatggtggca agcaggcgct ggagacggtc cagcggctgt tgccggtgct 4680
   gtgccaggcc cacggcttga ccccggagca ggtggtggcc atcgccagca atattggtgg 4740
   caagcaggcg ctggagacgg tgcaggcgct gttgccggtg ctgtgccagg cccacggctt 4800
   gacccctcag caggtggtgg ccatcgccag caatggcggc ggcaggccgg cgctggagag 4860
   cattgttgcc cagttatctc gccctgatcc ggcgttggcc gcgttgacca acgaccacct 4920
   cgtcgccttg gcctgcctcg gcgggcgtcc tgcgctggat gcagtgaaaa agggattggg 4980
   ggatcctatc agccgttccc agctggtgaa gtccgagctg gaggagaaga aatccgagtt 5040
   gaggcacaag ctgaagtacg tgccccacga gtacatcgag ctgatcgaga tcgcccggaa     5100
   cagcacccag gaccgtatcc tggagatgaa ggtgatggag ttcttcatga aggtgtacgg     5160
   ctacaggggc aagcacctgg gcggctccag gaagcccgac ggcgccatct acaccgtggg     5220
   ctcccccatc gactacggcg tgatcgtgga caccaaggcc tactccggcg gctacaacct     5280
   gcccatcggc caggccgacg aaatgcagag gtacgtggag gagaaccaga ccaggaacaa     5340
   gcacatcaac cccaacgagt ggtggaaggt gtacccctcc agcgtgaccg agttcaagtt     5400
   cctgttcgtg tccggccact tcaagggcaa ctacaaggcc cagctgacca ggctgaacca     5460
   catcaccaac tgcaacggcg ccgtgctgtc cgtggaggag ctcctgatcg gcggcgagat     5520
   gatcaaggcc ggcaccctga ccctggagga ggtgaggagg aagttcaaca acggcgagat     5580
   caacttcgcg gccgcttgat aactcgagcg atcctctaga cgagctcctc gagcctgcag     5640
   cagctgaagc tttaagatcc aatggcaagg accaagtgct ggaacttgtt ttgctttagc     5700
   agatctagat cgagctacct cgactttggc tgggacactt tcagtgagga caagaagctt     5760
   cagaagcgtg ctatcgaact caaccaggga cgtgcggcac aaatgggcat ccttgctctc     5820
   atggtgcacg aacagttggg agtctctatc cttccttaaa aatttaattt tcattagttg     5880
   cagtcactcc gctttggttt cacagtcagg aataacacta gctcgtcttc atatcctgca     5940
<210> 15
   <211> 6456
   <212> DNA
   <213> artificial sequence
<220>
   <223> plasmid vector: pCLS16353
<400> 15
   tcgcgcgttt cggtgatgac ggtgaaaacc tctgacacat gcagctcccg gagacggtca        60
   cagcttgtct gtaagcggat gccgggagca gacaagcccg tcagggcgcg tcagcgggtg       120
   ttggcgggtg tcggggctgg cttaactatg cggcatcaga gcagattgta ctgagagtgc       180
   accatatgat gcatccgtta acaccggtaa gcggccgcgc tagggataac agggtaatat       240
   tcaaaacgtc gtacgacgtt ttgacctgca ggaatctcgc ctattcatgg tgtataaaag       300
   ttcaacatcc aaagctagaa cttttggaaa gagaaagaat gtccgaatag ggcacggcgt       360
   gccgtattgt tggagtggac tagcagaaag tgaggaaggc acaggatgag tttcctcgag       420
   acacatagct tcagcgtcgt gtaggctagg cagaggtgag ttttctcgag acataccttc       480
   agcgtcgtct tcactgtcac agtcaactga cagtaatcgt tgatccggag agattcaaaa       540
   ttcaatctgt ttggacctgg ataagacaca agagcgacat cctgacatga acgccgtaaa       600
   cagcaaatcc tggttgaaca cgtatccttt tgggggcctc cagctacgac gctcgcccca      660
   gctggggctt ccttactata cacagcgcat atttcacggt tgccagaatt aattaagtag 720
   gcgcgccact agcgctgtca cgcgccaagc cgccaccatg ggcgatccta aaaagaaacg 780
   taaggtcatc gattacccat acgatgttcc agattacgct atcgatatcg ccgaccccat 840
   tcgttcgcgc acaccaagtc ctgcccgcga gcttctgccc ggaccccaac ccgatggggt 900
   tcagccgact gcagatcgtg gggtgtctcc gcctgccggc ggccccctgg atggcttgcc 960
   ggctcggcgg acgatgtccc ggacccggct gccatctccc cctgccccct cacctgcgtt 1020
   ctcggcgggc agcttcagtg acctgttacg tcagttcgat ccgtcacttt ttaatacatc 1080
   gctttttgat tcattgcctc ccttcggcgc tcaccataca gaggctgcca caggcgagtg 1140
   ggatgaggtg caatcgggtc tgcgggcagc cgacgccccc ccacccacca tgcgcgtggc 1200
   tgtcactgcc gcgcggcccc cgcgcgccaa gccggcgccg cgacgacgtg ctgcgcaacc 1260
   ctccgacgct tcgccggcgg cgcaggtgga tctacgcacg ctcggctaca gccagcagca 1320
   acaggagaag atcaaaccga aggttcgttc gacagtggcg cagcaccacg aggcactggt 1380
   cggccacggg tttacacacg cgcacatcgt tgcgttaagc caacacccgg cagcgttagg 1440
   gaccgtcgct gtcaagtatc aggacatgat cgcagcgttg ccagaggcga cacacgaagc 1500
   gatcgttggc gtcggcaaac agtggtccgg cgcacgcgct ctggaggcct tgctcacggt 1560
   ggcgggagag ttgagaggtc caccgttaca gttggacaca ggccaacttc tcaagattgc 1620
   aaaacgtggc ggcgtgaccg cagtggaggc agtgcatgca tggcgcaatg cactgacggg 1680
   tgccccgctc aacttgaccc cccagcaggt ggtggccatc gccagcaata atggtggcaa 1740
   gcaggcgctg gagacggtcc agcggctgtt gccggtgctg tgccaggccc acggcttgac 1800
   cccggagcag gtggtggcca tcgccagcaa tattggtggc aagcaggcgc tggagacggt 1860
   gcaggcgctg ttgccggtgc tgtgccaggc ccacggcttg accccggagc aggtggtggc 1920
   catcgccagc aatattggtg gcaagcaggc gctggagacg gtgcaggcgc tgttgccggt 1980
   gctgtgccag gcccacggct tgacccccca gcaggtggtg gccatcgcca gcaataatgg 2040
   tggcaagcag gcgctggaga cggtccagcg gctgttgccg gtgctgtgcc aggcccacgg 2100
   cttgaccccg gagcaggtgg tggccatcgc cagccacgat ggcggcaagc aggcgctgga 2160
   gacggtccag cggctgttgc cggtgctgtg ccaggcccac ggcttgaccc cggagcaggt 2220
   ggtggccatc gccagcaata ttggtggcaa gcaggcgctg gagacggtgc aggcgctgtt 2280
   gccggtgctg tgccaggccc acggcttgac cccccagcag gtggtggcca tcgccagcaa 2340
   taatggtggc aagcaggcgc tggagacggt ccagcggctg ttgccggtgc tgtgccaggc 2400
   ccacggcttg accccggagc aggtggtggc catcgccagc cacgatggcg gcaagcaggc 2460
   gctggagacg gtccagcggc tgttgccggt gctgtgccag gcccacggct tgaccccgga 2520
   gcaggtggtg gccatcgcca gcaatattgg tggcaagcag gcgctggaga cggtgcaggc 2580
   gctgttgccg gtgctgtgcc aggcccacgg cttgaccccc cagcaggtgg tggccatcgc 2640
   cagcaatggc ggtggcaagc aggcgctgga gacggtccag cggctgttgc cggtgctgtg 2700
   ccaggcccac ggcttgaccc cggagcaggt ggtggccatc gccagccacg atggcggcaa 2760
   gcaggcgctg gagacggtcc agcggctgtt gccggtgctg tgccaggccc acggcttgac 2820
   cccccagcag gtggtggcca tcgccagcaa taatggtggc aagcaggcgc tggagacggt 2880
   ccagcggctg ttgccggtgc tgtgccaggc ccacggcttg accccggagc aggtggtggc 2940
   catcgccagc aatattggtg gcaagcaggc gctggagacg gtgcaggcgc tgttgccggt 3000
   gctgtgccag gcccacggct tgacccccca gcaggtggtg gccatcgcca gcaatggcgg 3060
   tggcaagcag gcgctggaga cggtccagcg gctgttgccg gtgctgtgcc aggcccacgg 3120
   cttgaccccc cagcaggtgg tggccatcgc cagcaatggc ggtggcaagc aggcgctgga 3180
   gacggtccag cggctgttgc cggtgctgtg ccaggcccac ggcttgaccc ctcagcaggt 3240
   ggtggccatc gccagcaatg gcggcggcag gccggcgctg gagagcattg ttgcccagtt 3300
   atctcgccct gatccggcgt tggccgcgtt gaccaacgac cacctcgtcg ccttggcctg 3360
   cctcggcggg cgtcctgcgc tggatgcagt gaaaaaggga ttgggggatc ctatcagccg 3420
   ttcccagctg gtgaagtccg agctggagga gaagaaatcc gagttgaggc acaagctgaa 3480
   gtacgtgccc cacgagtaca tcgagctgat cgagatcgcc cggaacagca cccaggaccg 3540
   tatcctggag atgaaggtga tggagttctt catgaaggtg tacggctaca ggggcaagca 3600
   cctgggcggc tccaggaagc ccgacggcgc catctacacc gtgggctccc ccatcgacta 3660
   cggcgtgatc gtggacacca aggcctactc cggcggctac aacctgccca tcggccaggc 3720
   cgacgaaatg cagaggtacg tggaggagaa ccagaccagg aacaagcaca tcaaccccaa 3780
   cgagtggtgg aaggtgtacc cctccagcgt gaccgagttc aagttcctgt tcgtgtccgg 3840
   ccacttcaag ggcaactaca aggcccagct gaccaggctg aaccacatca ccaactgcaa 3900
   cggcgccgtg ctgtccgtgg aggagctcct gatcggcggc gagatgatca aggccggcac 3960
   cctgaccctg gaggaggtga ggaggaagtt caacaacggc gagatcaact tcgcggccga 4020
   ctgataactc gagcgatcct ctagacgagc tcctcgagcc tgcagcagct gaagctctag 4080
   cttgagctct cgagctacct cgactttggc tgggacactt tcagtgagga caagaagctt 4140
   cagaagcgtg ctatcgaact caaccaggga cgtgcggcac aaatgggcat ccttgctctc 4200
   atggtgcacg aacagttggg agtctctatc cttccttaaa aatttaattt tcattagttg 4260
   cagtcactcc gctttggttt cacagtcagg aataacacta gctcgtcttc agtttaaact 4320
   cactgactcg ctgcgctcgg tcgttcggct gcggcgagcg gtatcagctc actcaaaggc 4380
   ggtaatacgg ttatccacag aatcagggga taacgcagga aagacaattg cttataacac 4440
   gcgtactagt gctcgcgacg agatcttact taagcagtcg acaacctagg attagcgctc 4500
   cggtacctca aaacgtcgta cgacgttttg agctagggat aacagggtaa tatggatcca 4560
   agatatcaag aattcccatg tgagcaaaag gccagcaaaa ggccaggaac cgtaaaaagg 4620
   ccgcgttgct ggcgtttttc cataggctcc gcccccctga cgagcatcac aaaaatcgac 4680
   gctcaagtca gaggtggcga aacccgacag gactataaag ataccaggcg tttccccctg 4740
   gaagctccct cgtgcgctct cctgttccga ccctgccgct taccggatac ctgtccgcct 4800
   ttctcccttc gggaagcgtg gcgctttctc atagctcacg ctgtaggtat ctcagttcgg 4860
   tgtaggtcgt tcgctccaag ctgggctgtg tgcacgaacc ccccgttcag cccgaccgct 4920
   gcgccttatc cggtaactat cgtcttgagt ccaacccggt aagacacgac ttatcgccac 4980
   tggcagcagc cactggtaac aggattagca gagcgaggta tgtaggcggt gctacagagt 5040
   tcttgaagtg gtggcctaac tacggctaca ctagaaggac agtatttggt atctgcgctc 5100
   tgctgaagcc agttaccttc ggaaaaagag ttggtagctc ttgatccggc aaacaaacca 5160
   ccgctggtag cggtggtttt tttgtttgca agcagcagat tacgcgcaga aaaaaaggat 5220
   ctcaagaaga tcctttgatc ttttctacgg ggtctgacgc tcagtggaac gaaaactcac 5280
   gttaagggat tttggtcatg agattatcaa aaaggatctt cacctagatc cttttaaatt 5340
   aaaaatgaag ttttaaatca atctaaagta tatatgagta aacttggtct gacagttacc 5400
   aatgcttaat cagtgaggca cctatctcag cgatctgtct atttcgttca tccatagttg 5460
   cctgactccc cgtcgtgtag ataactacga tacgggaggg cttaccatct ggccccagtg 5520
   ctgcaatgat accgcgagac ccacgctcac cggctccaga tttatcagca ataaaccagc 5580
   cagccggaag ggccgagcgc agaagtggtc ctgcaacttt atccgcctcc atccagtcta 5640
   ttaattgttg ccgggaagct agagtaagta gttcgccagt taatagtttg cgcaacgttg 5700
   ttgccattgc tacaggcatc gtggtgtcac gctcgtcgtt tggtatggct tcattcagct 5760
   ccggttccca acgatcaagg cgagttacat gatcccccat gttgtgcaaa aaagcggtta 5820
   gctccttcgg tcctccgatc gttgtcagaa gtaagttggc cgcagtgtta tcactcatgg 5880
   ttatggcagc actgcataat tctcttactg tcatgccatc cgtaagatgc ttttctgtga 5940
   ctggtgagta ctcaaccaag tcattctgag aatagtgtat gcggcgaccg agttgctctt 6000
   gcccggcgtc aatacgggat aataccgcgc cacatagcag aactttaaaa gtgctcatca 6060
   ttggaaaacg ttcttcgggg cgaaaactct caaggatctt accgctgttg agatccagtt 6120
   cgatgtaacc cactcgtgca cccaactgat cttcagcatc ttttactttc accagcgttt 6180
   ctgggtgagc aaaaacagga aggcaaaatg ccgcaaaaaa gggaataagg gcgacacgga 6240
   aatgttgaat actcatactc ttcctttttc aatattattg aagcatttat cagggttatt 6300
   gtctcatgag cggatacata tttgaatgta tttagaaaaa taaacaaata ggggttccgc 6360
   gcacatttcc ccgaaaagtg ccacctgacg tctaagaaac cattattatc atgacattaa 6420
   cctataaaaa taggcgtatc acgaggccct ttcgtc 6456
<210> 16
   <211> 6474
   <212> DNA
   <213> artificial sequence
<220>
   <223> plasmid vector: pCLS16354
<400> 16
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgat gcatccgtta acaccggtaa gcggccgcgc tagggataac agggtaatat     240
   tcaaaacgtc gtacgacgtt ttgacctgca ggaatctcgc ctattcatgg tgtataaaag     300
   ttcaacatcc aaagctagaa cttttggaaa gagaaagaat gtccgaatag ggcacggcgt     360
   gccgtattgt tggagtggac tagcagaaag tgaggaaggc acaggatgag tttcctcgag     420
   acacatagct tcagcgtcgt gtaggctagg cagaggtgag ttttctcgag acataccttc     480
   agcgtcgtct tcactgtcac agtcaactga cagtaatcgt tgatccggag agattcaaaa     540
   ttcaatctgt ttggacctgg ataagacaca agagcgacat cctgacatga acgccgtaaa     600
   cagcaaatcc tggttgaaca cgtatccttt tgggggcctc cagctacgac gctcgcccca     660
   gctggggctt ccttactata cacagcgcat atttcacggt tgccagaatt aattaagtag     720
   gcgcgccact agcgctgtca cgcgccaagc cgccaccatg ggcgatccta aaaagaaacg     780
   taaggtcatc gataaggaga ccgccgctgc caagttcgag agacagcaca tggacagcat     840
   cgatatcgcc gaccccattc gttcgcgcac accaagtcct gcccgcgagc ttctgcccgg     900
   accccaaccc gatggggttc agccgactgc agatcgtggg gtgtctccgc ctgccggcgg     960
   ccccctggat ggcttgccgg ctcggcggac gatgtcccgg acccggctgc catctccccc    1020
   tgccccctca cctgcgttct cggcgggcag cttcagtgac ctgttacgtc agttcgatcc    1080
   gtcacttttt aatacatcgc tttttgattc attgcctccc ttcggcgctc accatacaga    1140
   ggctgccaca ggcgagtggg atgaggtgca atcgggtctg cgggcagccg acgccccccc    1200
   acccaccatg cgcgtggctg tcactgccgc gcggcccccg cgcgccaagc cggcgccgcg    1260
   acgacgtgct gcgcaaccct ccgacgcttc gccggcggcg caggtggatc tacgcacgct    1320
   cggctacagc cagcagcaac aggagaagat caaaccgaag gttcgttcga cagtggcgca    1380
   gcaccacgag gcactggtcg gccacgggtt tacacacgcg cacatcgttg cgttaagcca    1440
   acacccggca gcgttaggga ccgtcgctgt caagtatcag gacatgatcg cagcgttgcc 1500
   agaggcgaca cacgaagcga tcgttggcgt cggcaaacag tggtccggcg cacgcgctct 1560
   ggaggccttg ctcacggtgg cgggagagtt gagaggtcca ccgttacagt tggacacagg 1620
   ccaacttctc aagattgcaa aacgtggcgg cgtgaccgca gtggaggcag tgcatgcatg 1680
   gcgcaatgca ctgacgggtg ccccgctcaa cttgaccccc cagcaggtgg tggccatcgc 1740
   cagcaatggc ggtggcaagc aggcgctgga gacggtccag cggctgttgc cggtgctgtg 1800
   ccaggcccac ggcttgaccc cccagcaggt ggtggccatc gccagcaata atggtggcaa 1860
   gcaggcgctg gagacggtcc agcggctgtt gccggtgctg tgccaggccc acggcttgac 1920
   cccggagcag gtggtggcca tcgccagcca cgatggcggc aagcaggcgc tggagacggt 1980
   ccagcggctg ttgccggtgc tgtgccaggc ccacggcttg accccccagc aggtggtggc 2040
   catcgccagc aataatggtg gcaagcaggc gctggagacg gtccagcggc tgttgccggt 2100
   gctgtgccag gcccacggct tgacccccca gcaggtggtg gccatcgcca gcaatggcgg 2160
   tggcaagcag gcgctggaga cggtccagcg gctgttgccg gtgctgtgcc aggcccacgg 2220
   cttgaccccg gagcaggtgg tggccatcgc cagcaatatt ggtggcaagc aggcgctgga 2280
   gacggtgcag gcgctgttgc cggtgctgtg ccaggcccac ggcttgaccc cggagcaggt 2340
   ggtggccatc gccagccacg atggcggcaa gcaggcgctg gagacggtcc agcggctgtt 2400
   gccggtgctg tgccaggccc acggcttgac cccccagcag gtggtggcca tcgccagcaa 2460
   taatggtggc aagcaggcgc tggagacggt ccagcggctg ttgccggtgc tgtgccaggc 2520
   ccacggcttg accccccagc aggtggtggc catcgccagc aatggcggtg gcaagcaggc 2580
   gctggagacg gtccagcggc tgttgccggt gctgtgccag gcccacggct tgaccccgga 2640
   gcaggtggtg gccatcgcca gcaatattgg tggcaagcag gcgctggaga cggtgcaggc 2700
   gctgttgccg gtgctgtgcc aggcccacgg cttgaccccg gagcaggtgg tggccatcgc 2760
   cagcaatatt ggtggcaagc aggcgctgga gacggtgcag gcgctgttgc cggtgctgtg 2820
   ccaggcccac ggcttgaccc cccagcaggt ggtggccatc gccagcaatg gcggtggcaa 2880
   gcaggcgctg gagacggtcc agcggctgtt gccggtgctg tgccaggccc acggcttgac 2940
   cccccagcag gtggtggcca tcgccagcaa taatggtggc aagcaggcgc tggagacggt 3000
   ccagcggctg ttgccggtgc tgtgccaggc ccacggcttg accccggagc aggtggtggc 3060
   catcgccagc cacgatggcg gcaagcaggc gctggagacg gtccagcggc tgttgccggt 3120
   gctgtgccag gcccacggct tgaccccgga gcaggtggtg gccatcgcca gcaatattgg 3180
   tggcaagcag gcgctggaga cggtgcaggc gctgttgccg gtgctgtgcc aggcccacgg 3240
   cttgacccct cagcaggtgg tggccatcgc cagcaatggc ggcggcaggc cggcgctgga 3300
   gagcattgtt gcccagttat ctcgccctga tccggcgttg gccgcgttga ccaacgacca 3360
   cctcgtcgcc ttggcctgcc tcggcgggcg tcctgcgctg gatgcagtga aaaagggatt 3420
   gggggatcct atcagccgtt cccagctggt gaagtccgag ctggaggaga agaaatccga 3480
   gttgaggcac aagctgaagt acgtgcccca cgagtacatc gagctgatcg agatcgcccg 3540
   gaacagcacc caggaccgta tcctggagat gaaggtgatg gagttcttca tgaaggtgta 3600
   cggctacagg ggcaagcacc tgggcggctc caggaagccc gacggcgcca tctacaccgt 3660
   gggctccccc atcgactacg gcgtgatcgt ggacaccaag gcctactccg gcggctacaa 3720
   cctgcccatc ggccaggccg acgaaatgca gaggtacgtg gaggagaacc agaccaggaa 3780
   caagcacatc aaccccaacg agtggtggaa ggtgtacccc tccagcgtga ccgagttcaa 3840
   gttcctgttc gtgtccggcc acttcaaggg caactacaag gcccagctga ccaggctgaa 3900
   ccacatcacc aactgcaacg gcgccgtgct gtccgtggag gagctcctga tcggcggcga 3960
   gatgatcaag gccggcaccc tgaccctgga ggaggtgagg aggaagttca acaacggcga 4020
   gatcaacttc gcggccgact gataactcga gcgatcctct agacgagctc ctcgagcctg 4080
   cagcagctga agctctagct tgagctctcg agctacctcg actttggctg ggacactttc 4140
   agtgaggaca agaagcttca gaagcgtgct atcgaactca accagggacg tgcggcacaa 4200
   atgggcatcc ttgctctcat ggtgcacgaa cagttgggag tctctatcct tccttaaaaa 4260
   tttaattttc attagttgca gtcactccgc tttggtttca cagtcaggaa taacactagc 4320
   tcgtcttcag tttaaactca ctgactcgct gcgctcggtc gttcggctgc ggcgagcggt 4380
   atcagctcac tcaaaggcgg taatacggtt atccacagaa tcaggggata acgcaggaaa 4440
   gacaattgct tataacacgc gtactagtgc tcgcgacgag atcttactta agcagtcgac 4500
   aacctaggat tagcgctccg gtacctcaaa acgtcgtacg acgttttgag ctagggataa 4560
   cagggtaata tggatccaag atatcaagaa ttcccatgtg agcaaaaggc cagcaaaagg 4620
   ccaggaaccg taaaaaggcc gcgttgctgg cgtttttcca taggctccgc ccccctgacg 4680
   agcatcacaa aaatcgacgc tcaagtcaga ggtggcgaaa cccgacagga ctataaagat 4740
   accaggcgtt tccccctgga agctccctcg tgcgctctcc tgttccgacc ctgccgctta 4800
   ccggatacct gtccgccttt ctcccttcgg gaagcgtggc gctttctcat agctcacgct 4860
   gtaggtatct cagttcggtg taggtcgttc gctccaagct gggctgtgtg cacgaacccc 4920
   ccgttcagcc cgaccgctgc gccttatccg gtaactatcg tcttgagtcc aacccggtaa 4980
   gacacgactt atcgccactg gcagcagcca ctggtaacag gattagcaga gcgaggtatg 5040
   taggcggtgc tacagagttc ttgaagtggt ggcctaacta cggctacact agaaggacag 5100
   tatttggtat ctgcgctctg ctgaagccag ttaccttcgg aaaaagagtt ggtagctctt 5160
   gatccggcaa acaaaccacc gctggtagcg gtggtttttt tgtttgcaag cagcagatta 5220
   cgcgcagaaa aaaaggatct caagaagatc ctttgatctt ttctacgggg tctgacgctc 5280
   agtggaacga aaactcacgt taagggattt tggtcatgag attatcaaaa aggatcttca 5340
   cctagatcct tttaaattaa aaatgaagtt ttaaatcaat ctaaagtata tatgagtaaa 5400
   cttggtctga cagttaccaa tgcttaatca gtgaggcacc tatctcagcg atctgtctat 5460
   ttcgttcatc catagttgcc tgactccccg tcgtgtagat aactacgata cgggagggct 5520
   taccatctgg ccccagtgct gcaatgatac cgcgagaccc acgctcaccg gctccagatt 5580
   tatcagcaat aaaccagcca gccggaaggg ccgagcgcag aagtggtcct gcaactttat 5640
   ccgcctccat ccagtctatt aattgttgcc gggaagctag agtaagtagt tcgccagtta 5700
   atagtttgcg caacgttgtt gccattgcta caggcatcgt ggtgtcacgc tcgtcgtttg 5760
   gtatggcttc attcagctcc ggttcccaac gatcaaggcg agttacatga tcccccatgt 5820
   tgtgcaaaaa agcggttagc tccttcggtc ctccgatcgt tgtcagaagt aagttggccg 5880
   cagtgttatc actcatggtt atggcagcac tgcataattc tcttactgtc atgccatccg 5940
   taagatgctt ttctgtgact ggtgagtact caaccaagtc attctgagaa tagtgtatgc 6000
   ggcgaccgag ttgctcttgc ccggcgtcaa tacgggataa taccgcgcca catagcagaa 6060
   ctttaaaagt gctcatcatt ggaaaacgtt cttcggggcg aaaactctca aggatcttac 6120
   cgctgttgag atccagttcg atgtaaccca ctcgtgcacc caactgatct tcagcatctt 6180
   ttactttcac cagcgtttct gggtgagcaa aaacaggaag gcaaaatgcc gcaaaaaagg 6240
   gaataagggc gacacggaaa tgttgaatac tcatactctt cctttttcaa tattattgaa 6300
   gcatttatca gggttattgt ctcatgagcg gatacatatt tgaatgtatt tagaaaaata 6360
   aacaaatagg ggttccgcgc acatttcccc gaaaagtgcc acctgacgtc taagaaacca 6420
   ttattatcat gacattaacc tataaaaata ggcgtatcac gaggcccttt cgtc 6474
<210> 17
   <211> 5428
   <212> DNA
   <213> artificial sequence
<220>
   <223> plasmid vector: pCLS0003
<400> 17
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggag tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gctggctagc     900
   gtttaaactt aagcttggta ccgagctcgg atccactagt ccagtgtggt ggaattctgc     960
   agatatccag cacagtggcg gccgctcgag tctagagggc ccgtttaaac ccgctgatca    1020
   gcctcgactg tgccttctag ttgccagcca tctgttgttt gcccctcccc cgtgccttcc    1080
   ttgaccctgg aaggtgccac tcccactgtc ctttcctaat aaaatgagga aattgcatcg    1140
   cattgtctga gtaggtgtca ttctattctg gggggtgggg tggggcagga cagcaagggg    1200
   gaggattggg aagacaatag caggcatgct ggggatgcgg tgggctctat ggcttctgag    1260
   gcggaaagaa ccagctgggg ctctaggggg tatccccacg cgccctgtag cggcgcatta    1320
   agcgcggcgg gtgtggtggt tacgcgcagc gtgaccgcta cacttgccag cgccctagcg    1380
   cccgctcctt tcgctttctt cccttccttt ctcgccacgt tcgccggctt tccccgtcaa    1440
   gctctaaatc gggggctccc tttagggttc cgatttagtg ctttacggca cctcgacccc    1500
   aaaaaacttg attagggtga tggttcacgt agtgggccat cgccctgata gacggttttt    1560
   cgccctttga cgttggagtc cacgttcttt aatagtggac tcttgttcca aactggaaca    1620
   acactcaacc ctatctcggt ctattctttt gatttataag ggattttgcc gatttcggcc    1680
   tattggttaa aaaatgagct gatttaacaa aaatttaacg cgaattaatt ctgtggaatg    1740
   tgtgtcagtt agggtgtgga aagtccccag gctccccagc aggcagaagt atgcaaagca    1800
   tgcatctcaa ttagtcagca accaggtgtg gaaagtcccc aggctcccca gcaggcagaa    1860
   gtatgcaaag catgcatctc aattagtcag caaccatagt cccgccccta actccgccca    1920
   tcccgcccct aactccgccc agttccgccc attctccgcc ccatggctga ctaatttttt    1980
   ttatttatgc agaggccgag gccgcctctg cctctgagct attccagaag tagtgaggag    2040
   gcttttttgg aggcctaggc ttttgcaaaa agctcccggg agcttgtata tccattttcg    2100
   gatctgatca agagacagga tgaggatcgt ttcgcatgat tgaacaagat ggattgcacg    2160
   caggttctcc ggccgcttgg gtggagaggc tattcggcta tgactgggca caacagacaa    2220
   tcggctgctc tgatgccgcc gtgttccggc tgtcagcgca ggggcgcccg gttctttttg 2280
   tcaagaccga cctgtccggt gccctgaatg aactgcagga cgaggcagcg cggctatcgt 2340
   ggctggccac gacgggcgtt ccttgcgcag ctgtgctcga cgttgtcact gaagcgggaa 2400
   gggactggct gctattgggc gaagtgccgg ggcaggatct cctgtcatct caccttgctc 2460
   ctgccgagaa agtatccatc atggctgatg caatgcggcg gctgcatacg cttgatccgg 2520
   ctacctgccc attcgaccac caagcgaaac atcgcatcga gcgagcacgt actcggatgg 2580
   aagccggtct tgtcgatcag gatgatctgg acgaagagca tcaggggctc gcgccagccg 2640
   aactgttcgc caggctcaag gcgcgcatgc ccgacggcga ggatctcgtc gtgacccatg 2700
   gcgatgcctg cttgccgaat atcatggtgg aaaatggccg cttttctgga ttcatcgact 2760
   gtggccggct gggtgtggcg gaccgctatc aggacatagc gttggctacc cgtgatattg 2820
   ctgaagagct tggcggcgaa tgggctgacc gcttcctcgt gctttacggt atcgccgctc 2880
   ccgattcgca gcgcatcgcc ttctatcgcc ttcttgacga gttcttctga gcgggactct 2940
   ggggttcgaa atgaccgacc aagcgacgcc caacctgcca tcacgagatt tcgattccac 3000
   cgccgccttc tatgaaaggt tgggcttcgg aatcgttttc cgggacgccg gctggatgat 3060
   cctccagcgc ggggatctca tgctggagtt cttcgcccac cccaacttgt ttattgcagc 3120
   ttataatggt tacaaataaa gcaatagcat cacaaatttc acaaataaag catttttttc 3180
   actgcattct agttgtggtt tgtccaaact catcaatgta tcttatcatg tctgtatacc 3240
   gtcgacctct agctagagct tggcgtaatc atggtcatag ctgtttcctg tgtgaaattg 3300
   ttatccgctc acaattccac acaacatacg agccggaagc ataaagtgta aagcctgggg 3360
   tgcctaatga gtgagctaac tcacattaat tgcgttgcgc tcactgcccg ctttccagtc 3420
   gggaaacctg tcgtgccagc tgcattaatg aatcggccaa cgcgcgggga gaggcggttt 3480
   gcgtattggg cgctcttccg cttcctcgct cactgactcg ctgcgctcgg tcgttcggct 3540
   gcggcgagcg gtatcagctc actcaaaggc ggtaatacgg ttatccacag aatcagggga 3600
   taacgcagga aagaacatgt gagcaaaagg ccagcaaaag gccaggaacc gtaaaaaggc 3660
   cgcgttgctg gcgtttttcc ataggctccg cccccctgac gagcatcaca aaaatcgacg 3720
   ctcaagtcag aggtggcgaa acccgacagg actataaaga taccaggcgt ttccccctgg 3780
   aagctccctc gtgcgctctc ctgttccgac cctgccgctt accggatacc tgtccgcctt 3840
   tctcccttcg ggaagcgtgg cgctttctca tagctcacgc tgtaggtatc tcagttcggt 3900
   gtaggtcgtt cgctccaagc tgggctgtgt gcacgaaccc cccgttcagc ccgaccgctg 3960
   cgccttatcc ggtaactatc gtcttgagtc caacccggta agacacgact tatcgccact 4020
   ggcagcagcc actggtaaca ggattagcag agcgaggtat gtaggcggtg ctacagagtt 4080
   cttgaagtgg tggcctaact acggctacac tagaagaaca gtatttggta tctgcgctct 4140
   gctgaagcca gttaccttcg gaaaaagagt tggtagctct tgatccggca aacaaaccac     4200
   cgctggtagc ggtttttttg tttgcaagca gcagattacg cgcagaaaaa aaggatctca     4260
   agaagatcct ttgatctttt ctacggggtc tgacgctcag tggaacgaaa actcacgtta     4320
   agggattttg gtcatgagat tatcaaaaag gatcttcacc tagatccttt taaattaaaa     4380
   atgaagtttt aaatcaatct aaagtatata tgagtaaact tggtctgaca gttaccaatg     4440
   cttaatcagt gaggcaccta tctcagcgat ctgtctattt cgttcatcca tagttgcctg     4500
   actccccgtc gtgtagataa ctacgatacg ggagggctta ccatctggcc ccagtgctgc     4560
   aatgataccg cgagacccac gctcaccggc tccagattta tcagcaataa accagccagc     4620
   cggaagggcc gagcgcagaa gtggtcctgc aactttatcc gcctccatcc agtctattaa     4680
   ttgttgccgg gaagctagag taagtagttc gccagttaat agtttgcgca acgttgttgc     4740
   cattgctaca ggcatcgtgg tgtcacgctc gtcgtttggt atggcttcat tcagctccgg     4800
   ttcccaacga tcaaggcgag ttacatgatc ccccatgttg tgcaaaaaag cggttagctc     4860
   cttcggtcct ccgatcgttg tcagaagtaa gttggccgca gtgttatcac tcatggttat     4920
   ggcagcactg cataattctc ttactgtcat gccatccgta agatgctttt ctgtgactgg     4980
   tgagtactca accaagtcat tctgagaata gtgtatgcgg cgaccgagtt gctcttgccc     5040
   ggcgtcaata cgggataata ccgcgccaca tagcagaact ttaaaagtgc tcatcattgg     5100
   aaaacgttct tcggggcgaa aactctcaag gatcttaccg ctgttgagat ccagttcgat     5160
   gtaacccact cgtgcaccca actgatcttc agcatctttt actttcacca gcgtttctgg     5220
   gtgagcaaaa acaggaaggc aaaatgccgc aaaaaaggga ataagggcga cacggaaatg     5280
   ttgaatactc atactcttcc tttttcaata ttattgaagc atttatcagg gttattgtct     5340
   catgagcgga tacatatttg aatgtattta gaaaaataaa caaatagggg ttccgcgcac     5400
   atttccccga aaagtgccac ctgacgtc                                        5428

## Description

### FIELD OF THE SPECIFICATION

The specification relates to a method to select transformed cells. In particular, it relates to the use of a nuclease engineered to inactivate endogenous selectable marker which confers cell resistance or sensitivity to a toxic compound, especially the uridine-5'-monophosphate synthase (UMPS) gene or the nitrate reductase gene. The specification relates to methods of modifying genome of a cell, especially a diatom, comprising the present selection step. The present specification also relates to specific engineered nucleases, polynucleotides, vectors encoding thereof, kits and isolated cells comprising said nuclease.

### BACKGROUND

Applications of algal products range from simple biomass production for food, feed and fuels to valuable products such as cosmetics, pharmaceuticals, pigments, sugar polymers and food supplements.

As a particular group of microalgae, diatoms are one of the most ecologically successful unicellular phytoplankton on the planet, being responsible for approximately 20% of global carbon fixation, representing a major participant in the marine food web. One of the major potential commercial or technological applications of diatoms is the capacity to accumulate abundant amounts of lipid suitable for conversion to liquid fuels. Because of their high potential to produce large quantities of lipids and good growth efficiencies, they are considered as one of the best classes of algae for renewable biofuel production. As a particular group of microalgae, diatoms are the only major group of eukaryotic phytoplankton with a diplontic life history, in which all vegetative cells are diploid and meiosis produces short-lived, haploid gametes, suggesting an ancestral selection for a life history dominated by a duplicated (diploid) genome.

Although the genomes of several algal species have now been sequenced, very few genetic tools to explore microalgal genetics are available at this time, which considerably limits the use of these organisms for various biotechnological applications. The diploid genome organization and the unknown sexual reproduction properties in these model species impede classical approaches based on random mutagenesis and phenotypic selection. The generation of strains with a modulated gene expression resides mainly on the use of random gene over-expression and targeted gene-silencing system using RNA interference (RNAi) (Siaut, Heijde et al. 2007; De Riso, Raniello et al. 2009). Recently, the ability to perform targeted genomic manipulations within algal genome was facilitated by the use of homing endonuclease (WO 2012/017329).

Nevertheless, due to low transformation rates and the weak expression of transgenes, transformation methods require effective selection markers to discriminate successful transformed cells. However, only few publications refer to selection markers usable in Diatoms. Three antibiotics are shown to suppress the growth of cells and are used to select diatom transformed cells. (Dunahay, Jarvis et al. 1995; Zaslavskaia, Lippmeier et al. 2001) report the use of the neomycin phosphotransferase II (nptll), which inactivates G418 by phosphorylation, in *Cyclotella cryptica*, *Navicula saprophila and Phaeodactylum tricornutum* species. (Falciatore, Casotti et al. 1999; Zaslavskaia, Lippmeier et al. 2001) report the use of the Zeocin or Phleomycin resistance gene (Sh ble), acting by stochiometric binding, in *Phaeodactylum tricornutum* and *Cylindrotheca fusiformis* species. In (Zaslavskaia, Lippmeier et al. 2001), the use of N-acetyltransferase 1 gene (Natl) conferring the resistance to Nourseothricin by enzymatic acetylation is reported in *Phaeodactylum tricornutum* and *Thalassiosira pseudonana.*

Moreover, public concern about widespread use of antibiotic resistance markers has prompted the inventor to develop an alternative marker system which consists to use nucleases for targeting genes for which their inactivation allows selection of transformed cells. This method offers two advantages, firstly the identification of new selectable marker for the diatoms for which only few antibiotic and herbicide markers are available and secondly the selection of transformed cells without any antibiotic gene integrated into the genome, thus allowing generation of no genetically modified organisms. This selection requires the inactivation of both alleles for diploid strain and only one for haploid strain and can be considered by the ability of the nucleases to induce high frequency of targeted mutagenesis.

### SUMMARY

Described herein is a selection method based on the inactivation of a gene which confers resistance to a toxic substrate, in particular the uridine-5'-monophosphate synthase (UMPS) and the nitrate reductase gene. The inactivation of these genes has been shown to confer respectively the resistance to 5-Fluoroorotic acid (FOA) (Sakaguchi, Nakajima et al. 2011) and chlorate (Daboussi, Djeballi et al. 1989).

This method is particularly suitable for the selection of inactivated gene transformed cells by co-transformation of the nuclease targeting one of selectable marker genes with another protein of interest. The protein of interest can be a nuclease targeting a gene of interest to inactivate or a protein which increases the usability value of the algae in biotechnological applications. This co-transformation could be performed using multiple plasmids or using only one plasmid. Thus, we increase the proportion of transformed cells resistant to positive selection marker (5-FOA or Chlorate) containing the nuclease targeting the gene of interest. The delivery could be done by biolistic transformation, electroporation, micro-injection but also protein delivery using cell penetrating peptides, thus allowing the generation of no genetically modified organisms without transgene integration within the genome.

### DETAILED DESCRIPTION

Unless specifically defined herein, all technical and scientific terms used have the same meaning as commonly understood by a skilled artisan in the fields of gene therapy, biochemistry, genetics, and molecular biology.

The practice described herein will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

The specification relates to a selection method based on the inactivation of a selectable marker gene which confers resistance to a toxic substrate. This method comprises the step of introducing into a cell a nuclease capable of cleaving said selectable marker gene, and selecting cells resistant to said toxic compound. Particularly, the present specification relates to a method to select transformed diatom comprising:
(a) Selecting a selectable marker gene within a genome of a diatom which encodes a protein rendering a cell sensitive to a toxic substrate;
(b) Providing a TALE-nuclease which specifically recognizes and cleaves said selectable marker gene;
(c) Introducing said TALE-nuclease into diatoms such that said nuclease cleavage inactivates said selectable gene;
(d) Culturing said diatoms with said toxic substrate and;
(e) Selecting diatoms which are resistant to the toxic substrate,
   wherein said selectable marker gene and said toxic substrate are chosen from:
   - said selectable marker gene is the uridine-5'-monophosphate synthase (UMPS) gene and said toxic substrate is the 5-Fluoroorotic acid (5-FOA), and
   - said selectable marker gene is the nitrate reductase gene and said toxic substrate is chlorate;
   wherein said TALE-nuclease recognizes a target sequence selected from SEQ ID No:3 and SEQ ID No:4, or a target sequence having at least 90% identity with SEQ ID No:3 or SEQ ID No:4; and
   wherein said method further comprises: introducing into said diatom a polynucleotide comprising at least one homologous region to a part of said selectable marker gene such that said polynucleotide recombines with said selectable marker gene.

According to a preferred embodiment, the TALE-nuclease has an amino acid sequence selected from the group consisting of SEQ ID No: 5 to SEQ ID No:8, or has at least 80% identity with an amino acid sequence selected from SEQ ID No: 5 to SEQ ID No:8.

Selectable markers serve to eliminate unwanted elements. In particular, selectable marker gene is an endogenous gene which confers sensitivity to medium comprising a toxic substrate. Thus, inactivation of the selectable marker gene confers resistance to medium comprising toxic substrate. These markers are often toxic or otherwise inhibitory to replication under certain conditions. Consequently, it is possible to select cell comprising inactivated selectable marker gene. Selection of cells can also be obtained through the use of strains auxotropic for a particular metabolite. A point mutation or deletion in a gene required for amino acid synthesis or carbon source metabolism as examples can be used to select against strains when grown on media lacking the required nutrient. In most cases a defined "minimal" media is required for selection.

Said selectable marker is the Uridine 5'-monophosphate synthase gene (UMPS) encoding a protein which is involved in de novo synthesis of pyrimidine nucleotides and conversion of 5-Fluoroorotic acid (5-FOA) into the toxic compound 5-fluorouracil leading to cell death (Sakaguchi, Nakajima et al. 2011) or the nitrate reductase gene encoding a protein which confers sensitivity to chlorate (Daboussi, Djeballi et al. 1989). Said selectable marker can be homologous sequences of the different genes described above. Here, homology between protein or DNA sequences is defined in terms of shared ancestry. Two segments of DNA can have shared ancestry because of either a speciation event (orthologs) or a duplication event (paralogs). As described herein said cell is a diatom and said selectable marker genes is UMPS or nitrate reductase gene.

Inactivation of these selectable marker genes confers sensitivity to a toxic substrate. By inactivating a gene it is intended that the gene of interest is not expressed in a functional protein form. In a particular aspect described herein, the genetic modification of the method relies on the expression, in provided cells to engineer, of one nuclease such that said nuclease specifically catalyzes cleavage in one targeted gene thereby inactivating said targeted gene. The nucleic acid strand breaks caused by the nuclease are commonly repaired through the distinct mechanisms of homologous recombination or non-homologous end joining (NHEJ). However, NHEJ is an imperfect repair process that often results in changes to the DNA sequence at the site of the cleavage. Mechanisms involve rejoining of what remains of the two DNA ends through direct re-ligation (Critchlow and Jackson 1998) or via the so-called microhomology-mediated end joining (Ma, Kim et al. 2003). Repair via non-homologous end joining (NHEJ) often results in small insertions or deletions and can be used for the creation of specific gene knockouts. Said modification may be a substitution, deletion, or addition of at least one nucleotide.

Said nuclease can be a wild type or variant enzyme capable of catalyzing the hydrolysis (cleavage) of bonds between nucleic acids within a DNA or RNA molecule, preferably a DNA molecule. Particularly, said nuclease can be an endonuclease, more preferably a rare-cutting endonuclease which is highly specific, recognizing nucleic acid target sites ranging from 10 to 45 base pairs (bp) in length, usually ranging from 10 to 35 base pairs in length. The endonuclease according to the present invention recognizes and cleaves nucleic acid at specific polynucleotide sequences, further referred to as "target sequence". The rare-cutting endonuclease can recognize and generate a single- or double-strand break at specific polynucleotides sequences.

In an aspect, said rare-cutting endonuclease can be a Cas9 endonuclease. Indeed, recently a new genome engineering tool has been developed based on the RNA-guided Cas9 nuclease (Gasiunas, Barrangou et al. 2012; Jinek, Chylinski et al. 2012; Cong, Ran et al. 2013; Mali, Yang et al. 2013) from the type II prokaryotic CRISPR (Clustered Regularly Interspaced Short palindromic Repeats) adaptive immune system (see for review (Sorek, Lawrence et al. 2013)). The CRISPR Associated (Cas) system was first discovered in bacteria and functions as a defense against foreign DNA, either viral or plasmid. CRISPR-mediated genome engineering first proceeds by the selection of target sequence often flanked by a short sequence motif, referred as the proto-spacer adjacent motif (PAM). Following target sequence selection, a specific crRNA, complementary to this target sequence is engineered. Trans-activating crRNA (tracrRNA) required in the CRISPR type II systems paired to the crRNA and bound to the provided Cas9 protein. Cas9 acts as a molecular anchor facilitating the base pairing of tracRNA with cRNA (Deltcheva, Chylinski et al. 2011). In this ternary complex, the dual tracrRNA:crRNA structure acts as guide RNA that directs the endonuclease Cas9 to the cognate target sequence. Target recognition by the Cas9-tracrRNA:crRNA complex is initiated by scanning the target sequence for homology between the target sequence and the crRNA. In addition to the target sequence-crRNA complementarity, DNA targeting requires the presence of a short motif adjacent to the protospacer (protospacer adjacent motif - PAM). Following pairing between the dual-RNA and the target sequence, Cas9 subsequently introduces a blunt double strand break 3 bases upstream of the PAM motif (Garneau, Dupuis et al. 2010). In the present specification guide RNA can be designed to specifically target said selectable marker. Following the pairing between the guide RNA and the target sequence, Cas9 induce a cleavage (double strand break or single strand break) within selectable marker gene. By Cas9 is also meant an engineered endonuclease or a homologue of Cas9 or split Cas9 which is capable of processing target nucleic acid sequence. By "Split Cas9" is meant here a reduced or truncated form of a Cas9 protein or Cas9 variant, which comprises either a RuvC or HNH domain, but not both of these domains. Such "Split Cas9" can be used independently with guide RNA or in a complementary fashion, like for instance, one Split Cas9 providing a RuvC domain and another providing the HNH domain. Different split Cas9 may be used together having either RuvC and/or NHN domains.

Rare-cutting endonuclease can also be a homing endonuclease, also known under the name of meganuclease. Such homing endonucleases are well-known to the art (Stoddard 2005). Homing endonucleases are highly specific, recognizing DNA target sites ranging from 12 to 45 base pairs (bp) in length, usually ranging from 14 to 40 bp in length. The homing endonuclease may for example correspond to a LAGLIDADG endonuclease, to a HNH endonuclease, or to a GIY-YIG endonuclease. A homing endonuclease according to the present specification can be an I*-Crel* variant. A "variant" endonuclease, i.e. an endonuclease that does not naturally exist in nature and that is obtained by genetic engineering or by random mutagenesis can bind DNA sequences different from that recognized by wild-type endonucleases (see international application WO2006/097854).

Said rare-cutting endonuclease can be a modular DNA binding nuclease. By modular DNA binding nuclease is meant any fusion proteins comprising at least one catalytic domain of an endonuclease and at least one DNA binding domain or protein specifying a nucleic acid target sequence. The DNA binding domain is generally a RNA or DNA-binding domain formed by an independently folded polypeptide protein domain that contains at least one motif that recognizes double- or single-stranded polynucleotides. Many such polypeptides have been described in the art having the ability to bind specific nucleic acid sequences. Such binding domains often comprise, as non limiting examples, helix-turn helix domains, leucine zipper domains, winged helix domains, helix-loop-helix domains, HMG-box domains, Immunoglobin domains, B3 domain or engineered zinc finger domain.

The DNA binding domain may be derived from a Transcription Activator like Effector (TALE), wherein sequence specificity is driven by a series of 33-35 amino acids repeats originating from *Xanthomonas* or *Ralstonia* bacterial proteins. These repeats differ essentially by two amino acids positions that specify an interaction with a base pair (Boch, Scholze et al. 2009; Moscou and Bogdanove 2009). Each base pair in the DNA target is contacted by a single repeat, with the specificity resulting from the two variant amino acids of the repeat (the so-called repeat variable dipeptide, RVD). TALE binding domains may further comprise an N-terminal translocation domain responsible for the requirement of a first thymine base (T₀) of the targeted sequence and a C-terminal domain that containing a nuclear localization signals (NLS). A TALE nucleic acid binding domain generally corresponds to an engineered core TALE scaffold comprising a plurality of TALE repeat sequences, each repeat comprising a RVD specific to each nucleotides base of a TALE recognition site. In the present invention, each TALE repeat sequence of said core scaffold is made of 30 to 42 amino acids, more preferably 33 or 34 wherein two critical amino acids (the so-called repeat variable dipeptide, RVD) located at positions 12 and 13 mediates the recognition of one nucleotide of said TALE binding site sequence; equivalent two critical amino acids can be located at positions other than 12 and 13 specially in TALE repeat sequence taller than 33 or 34 amino acids long. Preferably, RVDs associated with recognition of the different nucleotides are HD for recognizing C, NG for recognizing T, NI for recognizing A, NN for recognizing G or A. In another embodiment, critical amino acids 12 and 13 can be mutated towards other amino acid residues in order to modulate their specificity towards nucleotides A, T, C and G and in particular to enhance this specificity. A TALE nucleic acid binding domain usually comprises between 8 and 30 TALE repeat sequences. More preferably, said core scaffold of the present invention comprises between 8 and 20 TALE repeat sequences; again more preferably 15 TALE repeat sequences. It can also comprise an additional single truncated TALE repeat sequence made of 20 amino acids located at the C-terminus of said set of TALE repeat sequences, i.e. an additional C-terminal half- TALE repeat sequence.

Other engineered DNA binding domains are modular base-per-base specific nucleic acid binding domains (MBBBD) (PCT/US2013/051783). Said MBBBD can be engineered, for instance, from the newly identified proteins, namely EAV36_BURRH, E5AW43_BURRH, E5AW45_BURRH and E5AW46_BURRH proteins from the recently sequenced genome of the endosymbiont fungi *Burkholderia Rhizoxinica* (Lackner, Moebius et al. 2011). MBBBD proteins comprise modules of about 31 to 33 amino acids that are base specific. These modules display less than 40 % sequence identity with *Xanthomonas* TALE common repeats, whereas they present more polypeptides sequence variability. When they are assembled together, these modular polypeptides can although target specific nucleic acid sequences in a quite similar fashion as *Xanthomonas* TALE-nucleases. According to the present specification, said DNA binding domain is an engineered MBBBD binding domain comprising between 10 and 30 modules, preferably between 16 and 20 modules. The different domains from the above proteins (modules, N and C-terminals) from Burkholderia and *Xanthomonas* are useful to engineer new proteins or scaffolds having binding properties to specific nucleic acid sequences. In particular, additional N-terminal and C-terminal domains of engineered MBBBD can be derived from natural TALE like AvrBs3, PthXo1, AvrHah1, PthA, Tal1c as examples. "TALE-nuclease" or "MBBBD-nuclease" refers to engineered proteins resulting from the fusion of a DNA binding domain typically derived from Transcription Activator like Effector proteins (TALE) or MBBBD binding domain, with an endonuclease catalytic domain. Such catalytic domain is preferably a nuclease domain and more preferably a domain having endonuclease activity, like for instance I-Tevl, ColE7, NucA and Fok-I. In a particular embodiment, said nuclease is a monomeric TALE-Nuclease or MBBBD-nuclease. A monomeric Nuclease is a nuclease that does not require dimerization for specific recognition and cleavage, such as the fusions of engineered DNA binding domain with the catalytic domain of I-Tevl described in WO2012138927. In another particular aspect described herein, said rare-cutting endonuclease is a dimeric TALE-nuclease or MBBBD-nuclease, preferably comprising a DNA binding domain fused to Fokl. Said dimeric nuclease comprises a first DNA binding nuclease capable of binding a target sequence comprising a part of the repeat sequence and a sequence adjacent thereto and a second DNA binding nuclease capable of binding a target sequence within the repeat sequence, such that the dimeric nuclease induces a cleavage event within the repeat sequence (see Figure 1). TALE-nuclease have been already described and used to stimulate gene targeting and gene modifications (Boch, Scholze et al. 2009; Moscou and Bogdanove 2009; Cermak, Doyle et al. 2010; Christian, Cermak et al. 2010). Such engineered TALE-nucleases are commercially available under the trade name TALEN™ (Cellectis, 8 rue de la Croix Jarry, 75013 Paris, France).

An additional catalytic domain can be further introduced into the cell with said nuclease to increase mutagenesis in order to enhance their capacity to inactivate targeted genes. In particular, said additional catalytic domain is a DNA end processing enzyme. Examples of DNA end-processing enzymes include 5-3' exonucleases, 3-5' exonucleases, 5-3' alkaline exonucleases, 5' flap endonucleases, helicases, hosphatase, hydrolases and template-independent DNA polymerases. Examples of such catalytic domain comprise of a protein domain or catalytically active derivate of the protein domain selected from the group consisting of hExol (EXO1_HUMAN), Yeast Exol (EXO1 YEAST), E.coli Exol, Human TREX2, Mouse TREX1, Human TREX1, Bovine TREX1, sae2 nuclease (CtBP-intracting protein (CtIP) homologue), Rat TREX1, TdT (terminal deoxynucleotidyl transferase) Human DNA2, Yeast DNA2 (DNA2_YEAST). In a preferred aspect described herein, said additional catalytic domain has a 3'-5'-exonuclease activity, and preferably said additional catalytic domain is TREX, even more preferably TREX2 catalytic domain (WO2012/058458). In another preferred aspect as described herein, said catalytic domain is encoded by a single chain TREX2 polypeptide. Said additional catalytic domain may be fused to a nuclease fusion protein or chimeric protein according to the invention optionally by a peptide linker.

Endonucleolytic breaks are known to stimulate the rate of homologous recombination. Thus, in another embodiment, the genetic modification step of the method further comprises a step of introduction into cells a donor matrix comprising at least a sequence homologous to a portion of the target nucleic acid sequence, such as the selectable marker gene, such that homologous recombination occurs between the target nucleic acid sequence and the donor matrix. In particular said donor matrix comprises first and second portions which are homologous to region 5' and 3' of the target nucleic acid sequence, respectively. Preferably, homologous equences of at least 50 bp, preferably more than 100 bp and more preferably more than 200 bp are used within said donor matrix. Therefore, the homologous sequence is preferably from 200 bp to 6000 bp, more preferably from 1000 bp to 2000 bp. Indeed, shared nucleic acid homologies are located in regions flanking upstream and downstream the site of the break and the nucleic acid sequence to be introduced should be located between the two arms.

In particular, said donor matrix successively comprises a first region of homology to sequences upstream of said cleavage, a sequence to inactivate one selectable marker gene and a second region of homology to sequences downstream of the cleavage. Said polynucleotide introduction step can be simultaneous, before or after the introduction or expression of said nuclease. Depending on the location of the target nucleic acid sequence wherein break event has occurred, such donor matrix can be used to knock-out a gene, e.g. when exogenous nucleic acid is located within the open reading frame of said gene, or to introduce new sequences or genes of interest. New sequences or gene of interest can encode a protein of interest, preferably a protein which increases the potential exploitation of algae by conferring them commercially desirable trait for various biotechnological applications or a nuclease which specifically targets a gene to inactivate within cell genome. Sequence insertions by using such donor matrix can be used to modify a targeted existing gene, by correction or replacement of said gene (allele swap as an example), or to up- or down-regulate the expression of the targeted gene (promoter swap as another example), said targeted gene correction or replacement.

The method can further comprise introducing another protein of interest into a cell. Preferably, the protein of interest is useful for increasing the usability and the commercial value of algae for various biotechnological applications. Said protein may be involved in the lipid metablolism. The protein of interest can also be a nuclease which can recognize and cleave a target sequence of interest. Resulting gene inactivation can increase the potential exploitation of algae by conferring them commercially desirable traits for various biotechnological applications, such as biofuel production.

Said protein of interest can be introduced as a transgene into the cell. Said transgenes encoding said protein of interest and nuclease cleaving selectable marker gene according to the present invention can be encoded by one or as different nucleic acid, preferably different vectors. Different transgenes can be included in one vector which comprises a nucleic acid sequence encoding ribosomal skip sequence such as a sequence encoding a 2A peptide. 2A peptides, which were identified in the Aphthovirus subgroup of picornaviruses, causes a ribosomal "skip" from one codon to the next without the formation of a peptide bond between the two amino acids encoded by the codons (see Donnelly et al., J. of General Virology 82: 1013-1025 (2001); Donnelly et al., J. of Gen. Virology 78: 13-21 (1997); Doronina et al., Mol. And. Cell. Biology 28(13): 4227-4239 (2008); Atkins et al., RNA 13: 803-810 (2007)). By "codon" is meant three nucleotides on an mRNA (or on the sense strand of a DNA molecule) that are translated by a ribosome into one amino acid residue. Thus, two polypeptides can be synthesized from a single, contiguous open reading frame within an mRNA when the polypeptides are separated by a 2A oligopeptide sequence that is in frame. Such ribosomal skip mechanisms are well known in the art and are known to be used by several vectors for the expression of several proteins encoded by a single messenger RNA. As an example 2A peptides have been used to express into the cell the nuclease cleaving the selectable marker gene, and the nuclease cleaving the gene of interest to inactivate, the DNA end-processing enzyme, the donor matrix or another transgene encoding a protein of interest.

### Delivery method

A variety of different methods are known for introducing protein of interest into cells. In various aspects described herein, said nuclease cleaving the selectable marker gene or other protein of interest can be encoded by a transgene, preferably comprised within a vector. In another embodiment, said protein of interest is encoded by RNA sequence. Said vectors or RNA sequence can be introduced into cell by, for example without limitation, electroporation, magnetophoresis. The latter is a nucleic acid introduction technology using the processes of magnetophoresis and nanotechnology fabrication of micro-sized linear magnets (Kuehnle et al., U. S. Patent No. 6,706,394; 2004; Kuehnle et al., U. S. Patent No. 5,516,670; 1996) that proved amenable to effective chloroplast engineering in freshwater *Chlamydomonas*, improving plastid transformation efficiency by two orders of magnitude over the state-of the-art of biolistics (Champagne et al., Magnetophoresis for pathway engineering in green cells. Metabolic engineering V: Genome to Product, Engineering Conferences International Lake Tahoe CA, Abstracts pp 76; 2004). Polyethylene glycol treatment of protoplasts is another technique that can be used to transform cells (Maliga 2004). The transformation methods can be coupled with one or more methods for visualization or quantification of nucleic acid introduction into cell. Also appropriate mixtures commercially available for protein transfection can be used to introduce protein in algae. More broadly, any means known in the art to allow delivery inside cells or subcellular compartments of agents/chemicals and molecules (proteins) can be used including liposomal delivery means, polymeric carriers, chemical carriers, lipoplexes, polyplexes, dendrimers, nanoparticles, emulsion, natural endocytosis or phagocytose pathway as examples. Direct introduction, such as microinjection of protein of interest or DNA in cell can be considered. In a more preferred aspect as described herein, said transformation construct is introduced into host cell by particle inflow gun bombardment or electroporation.

Said transgene or protein of interest can be introduced into the cell by using cell penetrating peptides (CPP). Said CPP can be associated with the transgene or protein of interest (named cargo molecule). This association can be covalent or non-covalent. CPPs can be subdivided into two main classes, the first requiring chemical linkage with the cargo and the second involving the formation of stable, non-covalent complexes. Said cargo molecule can be as non limiting example polynucleotides of either the DNA or RNA type, preferably polynucleotides encoding protein of interest, such as nuclease, marker molecule, proteins of interest useful to engineer the genetics of the algae, in particular, proteins involved in fatty acid metabolism, carbohydrate metabolism, genes associated with stress tolerance in growth conditions, and the like. Said cargo molecules can also be genes, expression cassettes, plasmids, sRNA, siRNA, miRNA shRNA, guide RNA of the CRISPR system and polypeptides such as protein of interest, nuclease, marker molecule.

Although definition of CPPs is constantly evolving, they are generally described as short peptides of less than 35 amino acids either derived from proteins or from chimeric sequences which are capable of transporting polar hydrophilic biomolecules across cell membrane in a receptor independent manner. CPP can be cationic peptides, peptides having hydrophobic sequences, amphipatic peptides, peptides having proline-rich and anti-microbial sequence, and chimeric or bipartite peptides (Pooga and Langel 2005). Cationic CPP can comprise multiple basic of cationic CPPs (e.g., arginine and/or lysine). Preferably, CCP are amphipathic and possess a net positive charge. CPPs are able to penetrate biological membranes, to trigger the movement of various biomolecules across cell membranes into the cytoplasm and to improve their intracellular routing, thereby facilitating interactions with the target. Examples of CPP can include Tat, a nuclear transcriptional activator protein which is a 101 amino acid protein required for viral replication by human immunodeficiency virus type 1 (HIV-1), penetratin, which corresponds to the third helix of the homeoprotein Antennapedia in *Drosophilia,* Kaposi fibroblast growth factor (FGF) signal peptide sequence, integrin β3 signal peptide sequence; MPG; pep-1; sweet arrow peptide, dermaseptins, transportan, pVEC, Human calcitonin, mouse prion protein (mPrPr) (REF: US2013/0065314).

### TALE-nucleases

In another aspect, the specification also relates to the nuclease disclosed here. In particular, the specification relates to a nuclease capable of recognizing a target sequence within the UMPS gene or the nitrate reductase gene, within the *P. tricornutum* UMPS gene (SEQ ID NO:1, GenBank: AB512669.1) or the *P. tricornutum* nitrate reductase gene (SEQ ID NO: 2 , GenBank: AY579336.1), in a preferred aspect a target sequence within the UMPS or nitrate reductase gene having at least 70%, preferably 80%, 85%, 90%; 95% identity with the nucleic acid sequence SEQ ID NO: 1 or 2. Said target sequence is selected from the group consisting of: SEQ ID NO: 3 and SEQ ID NO: 4, in a preferred aspect described herein, said target sequence has at least 90%, preferably 95% identity with the nucleic acid sequence selected from the group consisting of: SEQ ID NO: 3 and SEQ ID NO: 4.

Said nuclease is a TALE-nuclease. The specification relates to a TALE-nuclease having amino acid sequence selected from the group consisting of: SEQ ID NO: 5 to SEQ ID NO: 8, or said TALE-nuclease has at least 80%, preferably 85%, 90%; 95% identity with the amino acid sequence selected from the group consisting of: SEQ ID NO: 5 to SEQ ID NO: 8.

### Polynucleotides, vectors

The specification also describes the polynucleotides, in particular DNA or RNA encoding the nucleases previously described. These polynucleotides may be included in vectors, more particularly plasmids or virus, in view of being expressed in prokaryotic or eukaryotic cells. The polynucleotide may consist in an expression cassette or expression vector (e.g. a plasmid for introduction into a bacterial host cell, or a viral vector such as a baculovirus vector for transfection of an insect host cell, or a plasmid or viral vector such as a lentivirus for transfection of a mammalian host cell). In a particular aspect, the specification describes a polynucleotide comprising the nucleic acid sequence SEQ ID NO: 9 to SEQ ID NO: 12. Those skilled in the art will recognize that, in view of the degeneracy of the genetic code, considerable sequence variation is possible among these polynucleotide molecules. Preferably, the nucleic acid sequences of the present specification are codon-optimized for expression in algal cells, preferably for expression in diatom cells. Codon-optimization refers to the exchange in a sequence of interest of codons that are generally rare in highly expressed genes of a given species by codons that are generally frequent in highly expressed genes of such species, such codons encoding the amino acids as the codons that are being exchanged. In a preferred aspect, the polynucleotide has at least 70%, preferably at least 80%, more preferably at least 90 %, 95 % 97 % or 99 % sequence identity with nucleic acid sequence selected from the group consisting of SEQ ID NO: 9 to SEQ ID NO: 12.

### Isolated cells

Another aspect described herein relates to an isolated cell obtainable or obtained by the method described above, more specifically an isolated diatom. In particular, the specification relates to a diatom which comprises a nuclease capable of recognizing and cleaving a selectable marker gene chosen from the UMPS or nitrate reductase gene. In the frame of the present specification, "algae" or "algae cells" refer to different species of algae that can be used as host for selection method using nuclease. Algae are mainly photoautotrophs unified primarily by their lack of roots, leaves and other organs that characterize higher plants. Term "algae" groups, without limitation, several eukaryotic phyla, including the Rhodophyta (red algae), Chlorophyta (green algae), Phaeophyta (brown algae), Bacillariophyta (diatoms), Eustigmatophyta and dinoflagellates as well as the prokaryotic phylum Cyanobacteria (blue-green algae). The term "algae" includes for example algae selected from : *Amphora, Anabaena, Anikstrodesmis, Botryococcus, Chaetoceros, Chlamydomonas, Chlorella, Chlorococcum, Cyclotella, Cylindrotheca, Dunaliella, Emiliana, Euglena, Hematococcus, Isochrysis, Monochrysis, Monoraphidium, Nannochloris, Nannnochloropsis, Navicula, Nephrochloris, Nephroselmis, Nitzschia, Nodularia, Nostoc, Oochromonas, Oocystis, Oscillartoria, Pavlova, Phaeodactylum, Playtmonas, Pleurochrysis, Porhyra, Pseudoanabaena, Pyramimonas, Stichococcus, Synechococcus, Synechocystis, Tetraselmis, Thalassiosira,* and *Trichodesmium.*

Diatoms are unicellular phototrophs identified by their species-specific morphology of their amorphous silica cell wall, which vary from each other at the nanometer scale. Diatoms includes as non limiting examples: *Phaeodactylum, Fragilariopsis, Thalassiosira, Coscinodiscus, Arachnoidiscusm, Aster omphalus, Navicula, Chaetoceros, Chorethron, Cylindrotheca fusiformis, Cyclotella, Lampriscus, Gyrosigma, Achnanthes, Cocconeis, Nitzschia, Amphora, schizochytrium and Odontella.* Preferably diatoms may be from the species: *Thalassiosira pseudonana* or *Phaeodactylum tricornutum.*

### Kits

Another aspect described herein is a kit for algal cell selection comprising a nuclease which recognizes and cleaves a selectable marker as previously described. This kit more particularly comprises a nuclease capable of recognizing and cleaving a UMPS or nitrate reductase gene, optionally with the adequate toxic substrate for cell selection, such as chlorate or 5'FOA. In particular, the kit comprises a TALE-nuclease having at least 80%, more preferably at least 90 %, 95 % 97 % or 99 % sequence identity with amino acid sequence selected from the group consisting of SEQ ID NO: 5 to SEQ ID NO: 8. The kit may further comprise one or several components required to realize the selection method as described above.

### DEFINITIONS

In the specification above, a number of terms are used extensively. The following definitions are provided to facilitate understanding of the present specification.

As used herein, "a" or "an" may mean one or more than one.
- Amino acid residues in a polypeptide sequence are designated herein according to the one-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- As used herein, "nucleic acid" or "nucleic acid molecule" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Nucleic acids can be either single stranded or double stranded.
- By "gene" is meant the basic unit of heredity, consisting of a segment of DNA arranged in a linear manner along a chromosome, which codes for a specific protein or segment of protein. A gene typically includes a promoter, a 5' untranslated region, one or more coding sequences (exons), optionally introns, a 3' untranslated region. The gene may further comprise a terminator, enhancers and/or silencers.

By "genome" it is meant the entire genetic material contained in a cell such as nuclear genome, chloroplastic genome, mitochondrial genome.
- By "target sequence" is intended a polynucleotide sequence that can be processed by a rare-cutting endonuclease according to the present invention. These terms refer to a specific DNA location, preferably a genomic location in a cell, but also a portion of genetic material that can exist independently to the main body of genetic material such as plasmids, episomes, virus, transposons or in organelles such as mitochondria or chloroplasts as non-limiting examples. The nucleic acid target sequence is defined by the 5' to 3' sequence of one strand of said target.
- As used herein, the term transgene means a nucleic acid sequence (encoding, e.g., one or more polypeptides), which is partly or entirely heterologous, i.e., foreign, to the host cell into which it is introduced, or, is homologous to an endogenous gene of the host cell into which it is introduced, but which can be designed to be inserted, or can be inserted, into the cell genome in such a way as to alter the genome of the cell into which it is inserted (e.g., it is inserted at a location which differs from that of the natural gene or its insertion results in a knockout). A transgene can include one or more transcriptional regulatory sequences and any other nucleic acid, such as introns, that may be necessary for optimal expression of the selected nucleic acid encoding polypeptide. The polypeptide encoded by the transgene can be either not expressed, or expressed but not biologically active, in the algae or algal cells in which the transgene is inserted. Also, the transgene can be a sequence inserted in the genome for producing an interfering RNA. Most preferably, the transgene encodes a polypeptide useful for increasing the quantity and/or the quality of the lipid in the diatom.
- By "homologous" it is meant a sequence with enough identity to another one to lead to homologous recombination between sequences, more particularly having at least 95% identity, preferably 97% identity and more preferably 99%.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.
- By "vector" is intended to mean a nucleic acid molecule capable of transporting another nucleic acid to which it has been linked. A vector which can be used in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those skilled in the art and commercially available. Some useful vectors include, for example without limitation, pGEM13z. pGEMT and pGEMTEasy {Promega, Madison, WI); psTBluel (EMD Chemicals Inc. San Diego, CA); and pcDNA3.1, pCR4- TOPO, pCR-TOPO-II, pCRBlunt-II-TOPO (Invitrogen, Carlsbad, CA). Preferably said vectors are expression vectors, wherein the sequence(s) encoding the rare-cutting endonuclease of the invention is placed under control of appropriate transcriptional and translational control elements to permit production or synthesis of said rare-cutting endonuclease. Therefore, said polynucleotide is comprised in an expression cassette. More particularly, the vector comprises a replication origin, a promoter operatively linked to said polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said rare-cutting endonuclease is a heterodimer, the two polynucleotides encoding each of the monomers are included in two vectors to avoid intraplasmidic recombination events. In another embodiment the two polynucleotides encoding each of the monomers are included in one vector which is able to drive the expression of both polynucleotides, simultaneously. In some embodiments, the vector for the expression of the rare-cutting endonucleases according to the invention can be operably linked to an algal-specific promoter. In some embodiments, the algal-specific promoter is an inducible promoter. In some embodiments, the algal-specific promoter is a constitutive promoter. Promoters that can be used include, for example without limitation, a Pptca1 promoter (the CO2 responsive promoter of the chloroplastic carbonic anyhydrase gene, ptca1, from *P. tricornutum),* a NITI promoter, an AMTI promoter, an AMT2 promoter, an AMT4 promoter, a RHI promoter, a cauliflower mosaic virus 35S promoter, a tobacco mosaic virus promoter, a simian virus 40 promoter, a ubiquitin promoter, a PBCV-I VP54 promoter, or functional fragments thereof, or any other suitable promoter sequence known to those skilled in the art. In another more preferred embodiment according to the present invention the vector is a shuttle vector, which can both propagate in *E. coli* (the construct containing an appropriate selectable marker and origin of replication) and be compatible for propagation or integration in the genome of the selected algae.
- The term "promoter" as used herein refers to a minimal nucleic acid sequence sufficient to direct transcription of a nucleic acid sequence to which it is operably linked. The term "promoter" is also meant to encompass those promoter elements sufficient for promoter-dependent gene expression controllable for cell-type specific expression, tissue specific expression, or inducible by external signals or agents; such elements may be located in the 5' or 3' regions of the naturally-occurring gene.

By "inducible promoter" it is mean a promoter that is transcriptionally active when bound to a transcriptional activator, which in turn is activated under a specific condition(s), e.g., in the presence of a particular chemical signal or combination of chemical signals that affect binding of the transcriptional activator, e.g., CO₂ or NO₂, to the inducible promoter and/or affect function of the transcriptional activator itself.

The term "host cell" refers to a cell that is transformed using the methods of the invention. In general, host cell as used herein means an algal cell into which a nucleic acid target sequence has been modified.

By "mutagenesis" is understood the elimination or addition of at least one given DNA fragment (at least one nucleotide) or sequence, bordering the recognition sites of rare-cutting endonuclease.

By "NHEJ" (non-homologous end joining) is intended a pathway that repairs double-strand breaks in DNA in which the break ends are ligated directly without the need for a homologous template. NHEJ comprises at least two different processes. Mechanisms involve rejoining of what remains of the two DNA ends through direct re-ligation {Critchlow, 1998 #17} or via the so-called microhomology-mediated end joining (Akopian, He et al. 2003) that results in small insertions or deletions and can be used for the creation of specific gene knockouts.

The term "Homologous recombination" refers to the conserved DNA maintenance pathway involved in the repair of DSBs and other DNA lesions. In gene targeting experiments, the exchange of genetic information is promoted between an endogenous chromosomal sequence and an exogenous DNA construct. Depending of the design of the targeted construct, genes could be knocked out, knocked in, replaced, corrected or mutated, in a rational, precise and efficient manner. The process requires homology between the targeting construct and the targeted locus. Preferably, homologous recombination is performed using two flanking sequences having identity with the endogenous sequence in order to make more precise integration as described in WO9011354.

The above written description of the invention provides a manner and process of making and using it such that any person skilled in this art is enabled to make and use the same, this enablement being provided for the subject matter of the appended claims, which make up a part of the original description.

As used above, the phrases "selected from the group consisting of", "chosen from" and the like include mixtures of the specified materials.

Where a numerical limit or range is stated herein, the endpoints are included. Also, all values and sub-ranges within a numerical limit or range are specifically included as if explicitly written out.

The above specification is presented to enable a person skilled in the art to make and use the invention, and is provided in the context of a particular application and its requirements.

### EXAMPLES

### Example 1: New method for selection of diatom-transformed cells using a TALEN targeting the UMPS gene and conferring a resistance to 5-FOA

Due to the very low transformation efficacy 10⁻⁸, the delivery of a protein encoding plasmid in the marine diatom *Phaeodactylum tricornutum* is mediated by co-transformation with antibiotic selectable marker.

Here, we propose the use of new selectable method which consists to the co-transformation of a plasmid encoding the protein of interest and plasmids encoding a TALEN targeting the gene UMPS encoding the key enzyme in the synthesis of pyrimidines: Uridine-5'-monophosphate synthase. The mutagenic events induced by this TALEN could lead to gene inactivation which has been previously reported to confer a resistance to 5-Fluoroorotic acid (5-FOA) as it has been previously reported (Sakaguchi, Nakajima et al. 2011). For that, a UMPS_TALEN (SEQ ID NO: 5 and SEQ ID NO: 6) encoded by the pCLS20603 (SEQ ID NO: 13) and pCLS20604 (SEQ ID NO: 14) plasmids designed to cleave the DNA sequence 5' -TTTAGTCTGTCTCTAGGTGTTCTCAAATTCGGCTCTTTTGTGCTGAAAA- 3' (SEQ ID NO: 3) were used. The diatoms transformed by this TALEN will be selected on 5-FOA medium according with the conditions described in (Sakaguchi, Nakajima et al. 2011).

### Materials and methods

### Culture conditions

*Phaeodactylum tricornutum* Bohlin clone CCMP2561 was grown in filtered Guillard's f/2 medium without silica (40°/°° w/v Sigma Sea Salts S9883, supplemented with 1X Guillard's f/2 marine water enrichment solution (Sigma G0154) in a Sanyo incubator (model MLR-351) at a constant temperature (20 +/- 0.5 °C). The incubator is equipped with white cold neon light tubes that produce an illumination of about 120 µmol photons m⁻² s⁻¹ and a photoperiod of 12h light : 12h darkness (illumination period from 9AM to 9PM).

### Genetic transformation

5.10⁷ cells were collected from exponentially growing liquid cultures (concentration about 10⁶ cells/ ml) by centrifugation (3000 rpm for 10 minutes at 20°C). The supernatant was discarded and the cell pellet resuspended in 500µl of fresh f/2 medium. The cell suspension was then spread on the center one-third of a 10 cm 1% agar plate containing 20°/°° sea salts supplemented with f/2 solution without silica. Two hours later, transformation was carried out using the microparticle bombardment (Biolistic PDS-1000/He Particle Delivery System (BioRad)). The protocol is adapted from (Falciatore, Casotti et al. 1999) and (Apt, Kroth-Pancic et al. 1996) with minor modifications. Briefly, M17 tungstene particles (1.1µm diameter, BioRad) were coated with 6µg of total amount of DNA containing 3µg of each monomer of TALENs (pCLS20603 and pCLS20604), using 1.25M CaCl2 and 20mM spermidine according to the manufacturer's instructions. As negative control, beads were coated with a DNA mixture containing 6µg empty vector (pCLS0003) (SEQ ID NO: 17). Agar plates with the diatoms to be transformed were positioned at 7.5cm from the stopping screen within the bombardment chamber (target shelf on position two). A burst pressure of 1550 psi and a vacuum of 25Hg/in were used. After bombardment, plates were incubated for 48 hours with a 12h light: 12h dark photoperiod.

### Selection

Two days post transformation, bombarded cells were gently scrapped with 700µl of f/2 medium without silica and spread on two 10 cm 1% agar plates (20°/°° sea salts supplemented with f/2 medium without silica) containing 5-FOA. Plates were then placed in the incubator under a 12h light: 12h darkness cycle for at least three weeks.

### Characterization

Resistant colonies were picked and dissociated in 20µl of lysis buffer (1% TritonX-100, 20mM Tris-HCI pH8, 2mM EDTA) in an eppendorf tube. Tubes were vortexed for at least 30 sec and then kept on ice for 15 min. After heating for 10 min at 85°C, tubes were cooled down at RT and briefly centrifuged to pellet cells debris. Supernatants were used immediately or stocked at 4°C. 5µl of a 1:5 dilution in milliQ H2O of the supernatants, were used for each PCR reaction. The UMPS target will be amplified using a 1:5 dilution of the lysis colony with specific primers and sequenced to identify the nature of mutagenic event.

### Results

The transformation of diatoms with plasmids encoding TALEN would lead to the UMPS gene inactivation conferring the ability to grow on medium supplemented with 5-FOA.

### Example 2: New method for selection of diatom-transformed cells using a TALEN targeting the Nitrate reductase gene and conferring a resistance to chlorate

Due to the very low transformation efficacy 10⁻⁸, the delivery of a protein encoding plasmid in the marine diatom *Phaeodactylum tricornutum* is mediated by co-transformation with antibiotic selectable marker.

Here, we propose the use of new selectable method which consists to the co-transformation of a plasmid encoding the protein of interest and plasmids encoding a TALEN targeting the gene NR encoding one key enzyme in the Nitrate metabolism: Nitrate reductase. The mutagenic events induced by this TALEN could lead to gene inactivation which has been previously reported to confer a resistance to Chlorate as it has been previously reported (Daboussi, Djeballi et al. 1989). For that, a NR_TALEN (SEQ ID NO: 7 and SEQ ID NO: 8) encoded by the pCLS16353 (SEQ ID NO: 15) and pCLS16354 (SEQ ID NO: 16) plasmids designed to cleave the DNA sequence 5'-TGAAGCAGCATCGATTTATTACGCCGTCCTCGTTGCATTACGTACGCAA- 3' (SEQ ID NO: 4) were used. The diatoms transformed by this TALEN will be selected on chlorate medium according with the conditions described in (Daboussi, Djeballi et al. 1989).

### Materials and methods

### Culture conditions

*Phaeodactylum tricornutum* Bohlin clone CCMP2561 was grown in filtered Guillard's f/2 medium without silica (40‰ w/v Sigma Sea Salts S9883, supplemented with 1X Guillard's f/2 marine water enrichment solution (Sigma G0154) in a Sanyo incubator (model MLR-351) at a constant temperature (20 +/- 0.5 °C). The incubator is equipped with white cold neon light tubes that produce an illumination of about 120 µmοl photons m⁻² s⁻¹ and a photoperiod of 12h light : 12h darkness (illumination period from 9AM to 9PM).

### Genetic transformation

5.10⁷ cells were collected from exponentially growing liquid cultures (concentration about 10⁶ cells/ ml) by centrifugation (3000 rpm for 10 minutes at 20°C). The supernatant was discarded and the cell pellet resuspended in 500µl of fresh f/2 medium. The cell suspension was then spread on the center one-third of a 10 cm 1% agar plate containing 20‰ sea salts supplemented with f/2 solution without silica. Two hours later, transformation was carried out using the microparticle bombardment (Biolistic PDS-1000/He Particle Delivery System (BioRad)). The protocol is adapted from (Falciatore, Casotti et al. 1999) and (Apt, Kroth-Pancic et al. 1996) with minor modifications. Briefly, M17 tungstene particles (1.1µm diameter, BioRad) were coated with 6µg of total amount of DNA containing 3µg of each monomer of TALENs (pCLS16353 and pCLS16354), using 1.25M CaCl2 and 20mM spermidine according to the manufacturer's instructions. As negative control, beads were coated with a DNA mixture containing 6µg empty vector (pCLS0003) (SEQ ID NO: 17). Agar plates with the diatoms to be transformed were positioned at 7.5cm from the stopping screen within the bombardment chamber (target shelf on position two). A burst pressure of 1550 psi and a vacuum of 25Hg/in were used. After bombardment, plates were incubated for 48 hours with a 12h light: 12h dark photoperiod.

### Selection

Two days post transformation, bombarded cells were gently scrapped with 700µl of f/2 medium without silica and spread on two 10 cm 1% agar plates (20°/°° sea salts supplemented with f/2 medium without silica) containing Chlorate. Plates were then placed in the incubator under a 12h light: 12h darkness cycle for at least three weeks.

### Characterization

Resistant colonies were picked and dissociated in 20µl of lysis buffer (1% TritonX-100, 20mM Tris-HCI pH8, 2mM EDTA) in an eppendorf tube. Tubes were vortexed for at least 30 sec and then kept on ice for 15 min. After heating for 10 min at 85°C, tubes were cooled down at RT and briefly centrifuged to pellet cells debris. Supernatants were used immediately or stocked at 4°C. 5µl of a 1:5 dilution in milliQ H2O of the supernatants, were used for each PCR reaction. The UMPS target will be amplified using a 1:5 dilution of the lysis colony with specific primers and sequenced to identify the nature of mutagenic event.

### Results

The transformation of diatoms with plasmids encoding TALEN would lead to the NR gene inactivation conferring the ability to grow on medium supplemented with chlorate.

### REFERENCES

Akopian, A., J. He, et al. (2003). "Chimeric recombinases with designed DNA sequence recognition." Proc Natl Acad Sci U S A 100(15): 8688-91.
Apt, K. E., P. G. Kroth-Pancic, et al. (1996). "Stable nuclear transformation of the diatom Phaeodactylum tricornutum." Mol Gen Genet 252(5): 572-9.
Boch, J., H. Scholze, et al. (2009). "Breaking the code of DNA binding specificity of TAL-type III effectors." Science 326(5959): 1509-12.
Cermak, T., E. L. Doyle, et al. (2010). "Efficient design and assembly of custom TALEN and other TAL effector-based constructs for DNA targeting." Nucleic Acids Res 39(12): e82.
Christian, M., T. Cermak, et al. (2010). "Targeting DNA double-strand breaks with TAL effector nucleases." Genetics 186(2): 757-61.
Cong, L., F. A. Ran, et al. (2013). "Multiplex genome engineering using CRISPR/Cas systems." Science 339(6121): 819-23.
Critchlow, S. E. and S. P. Jackson (1998). "DNA end-joining: from yeast to man." Trends Biochem Sci 23(10): 394-8.
Daboussi, M. J., A. Djeballi, et al. (1989). "Transformation of seven species of filamentous fungi using the nitrate reductase gene of Aspergillus nidulans." Curr Genet 15(6): 453-6.
De Riso, V., R. Raniello, et al. (2009). "Gene silencing in the marine diatom Phaeodactylum tricornutum." Nucleic Acids Res 37(14): e96.
Deltcheva, E., K. Chylinski, et al. (2011). "CRISPR RNA maturation by trans-encoded small RNA and host factor RNase III."Nature 471(7340): 602-7.
Dunahay, T. G., E. E. Jarvis, et al. (1995). "Genetic transformation of the diatoms Cyclotella Cryptica and Navicula Saprophila." Journal of Phycology 31(6): 1004-1012.
Falciatore, A., R. Casotti, et al. (1999). "Transformation of Nonselectable Reporter Genes in Marine Diatoms." Mar Biotechnol (NY) 1(3): 239-251.
Garneau, J. E., M. E. Dupuis, et al. (2010). "The CRISPR/Cas bacterial immune system cleaves bacteriophage and plasmid DNA." Nature 468(7320): 67-71.
Gasiunas, G., R. Barrangou, et al. (2012). "Cas9-crRNA ribonucleoprotein complex mediates specific DNA cleavage for adaptive immunity in bacteria." Proc Natl Acad Sci U S A 109(39): E2579-86.
Jinek, M., K. Chylinski, et al. (2012). "A programmable dual-RNA-guided DNA endonuclease in adaptive bacterial immunity." Science 337(6096): 816-21.
Lackner, G., N. Moebius, et al. (2011). "Complete genome sequence of Burkholderia rhizoxinica, an Endosymbiont of Rhizopus microsporus." J Bacteriol 193(3): 783-4.
Ma, J. L., E. M. Kim, et al. (2003). "Yeast Mre11 and Rad1 proteins define a Ku-independent mechanism to repair double-strand breaks lacking overlapping end sequences." Mol Cell Biol 23(23): 8820-8.
Mali, P., L. Yang, et al. (2013). "RNA-guided human genome engineering via Cas9." Science 339(6121): 823-6.
Maliga, P. (2004). "Plastid transformation in higher plants." Annu Rev Plant Biol 55: 289-313. Moscou, M. J. and A. J. Bogdanove (2009). "A simple cipher governs DNA recognition by TAL effectors." Science 326(5959): 1501.
Pooga, M. and U. Langel (2005). "Synthesis of cell-penetrating peptides for cargo delivery." Methods Mol Biol 298: 77-89.
Sakaguchi, T., K. Nakajima, et al. (2011). "Identification of the UMP synthase gene by establishment of uracil auxotrophic mutants and the phenotypic complementation system in the marine diatom Phaeodactylum tricornutum." Plant Physiol 156(1): 78-89.
Siaut, M., M. Heijde, et al. (2007). "Molecular toolbox for studying diatom biology in Phaeodactylum tricornutum." Gene 406(1-2): 23-35.
Sorek, R., C. M. Lawrence, et al. (2013). "CRISPR-mediated Adaptive Immune Systems in Bacteria and Archaea." Annu Rev Biochem.
Stoddard, B. L. (2005). "Homing endonuclease structure and function." Q Rev Biophys 38(1): 49-95. Zaslavskaia, L. A., J. C. Lippmeier, et al. (2001). "Trophic conversion of an obligate photoautotrophic organism through metabolic engineering." Science 292(5524): 2073-5.

## Claims

1. A method of modifying a diatom comprising:
(a) Selecting a selectable marker gene within the genome of a diatom which encodes a protein rendering a cell sensitive to a toxic substrate;
(b) Providing a TALE-nuclease which specifically recognizes and cleaves a target sequence within said selectable marker gene;
(c) Introducing said TALE-nuclease into diatoms such that said nuclease cleavage inactivates said selectable marker gene;
(d) Culturing said diatoms with said toxic substrate and;
(e) Selecting diatoms which are resistant to the toxic substrate,
wherein said selectable marker gene and said toxic substrate are chosen from:
- said selectable marker gene is the uridine-5'-monophosphate synthase (UMPS) gene and said toxic substrate is the 5-Fluoroorotic acid (5-FOA), and
- said selectable marker gene is the nitrate reductase gene and said toxic substrate is chlorate,
wherein said TALE-nuclease recognizes a target sequence selected from SEQ ID No:3 and SEQ ID No:4, or a target sequence having at least 90% identity with SEQ ID No:3 or SEQ ID No:4; and
wherein said method further comprises: introducing into said diatom a polynucleotide comprising at least one homologous region to a part of said selectable marker gene such that said polynucleotide recombines with said selectable marker gene.

2. The method of claim 1, wherein said TALE-nuclease has an amino acid sequence selected from the group consisting of SEQ ID No: 5 to SEQ ID No:8, or has at least 80% identity with an amino acid sequence selected from SEQ ID No: 5 to SEQ ID No:8.

3. The method of claim 1 comprising in step c) transforming said diatom with a polynucleotide encoding said TALE-nuclease and expressing said TALE-nuclease into the cell.

4. The method of claim 1 wherein said diatom is selected from the group consisting of: *Thalassiosira pseudonana* or *Phaeodactylum tricornutum*

5. The method according to any one of claims 1 to 4 wherein said selectable marker gene is the uridine-5'-monophosphate synthase (UMPS) gene and said toxic substrate is the 5-Fluoroorotic acid (5-FOA).

6. The method according to any one of claims 1 to 4 wherein said selectable marker gene is the nitrate reductase gene and said toxic substrate is chlorate.

7. The method according to any one of claims 1 to 6 further comprising introducing at least another protein of interest into said diatom.

8. The method of claim 7 wherein said another protein of interest is a nuclease capable of recognizing and cleaving a target sequence of interest.

9. The method according to any one of claims 1 to 8, wherein the selection of transformed diatoms is made without any antibiotic selectable marker gene integrated into the genome.

10. A diatom obtainable by a method according to any one of claims 1-9, comprising a TALE-nuclease which recognizes a target sequence within a gene selected from the UMPS gene (SEQ ID NO:1) and the nitrate reductase gene (SEQ ID NO:2), wherein the TALE-nuclease has an amino acid sequence selected from the group consisting of SEQ ID No: 5 to SEQ ID No:8, or has at least 80% identity with an amino acid sequence selected from SEQ ID No: 5 to SEQ ID No:8; and wherein the TALE-nuclease recognizes a target sequence having at least 90% identity with SEQ ID No:3 or SEQ ID No:4.

## Patentansprüche

1. Verfahren zur Modifizierung einer Kieselalge, welches umfasst:
(a) das Auswählen eines selektierbaren Marker-Gens innerhalb des Genoms einer Kieselalge, welches für ein Protein kodiert, das eine Zelle gegenüber einem toxischen Substrat sensibilisiert;
(b) das Bereitstellen einer TALE-Nuklease, die eine Zielsequenz innerhalb des selektierbaren Marker-Gens selektiv erkennt und schneidet;
(c) das Einführen der TALE-Nuklease in Kieselalgen, so dass der Schnitt durch die Nuklease das selektierbare Marker-Gen inaktiviert;
(d) das Kultivieren der Kieselalgen mit dem toxischen Substrat; und
(e) das Selektieren von Kieselalgen, die gegen das toxische Substrat resistent sind,
wobei das selektierbare Marker-Gen und das toxische Substrat ausgewählt sind aus:
- das selektierbare Marker-Gen ist das Uridin-5'-Monophosphat-Synthase-Gen (UMPS-Gen), und das toxische Substrat ist 5-Fluororotsäure (5-FOA), und
- das selektierbare Marker-Gen ist das Nitrat-Reduktase-Gen, und das toxische Substrat ist Chlorat,
wobei die TALE-Nuklease eine Zielsequenz, die aus SEQ ID NO:3 und SEQ ID NO:4 ausgewählt ist, oder eine Zielsequenz, die mindestens 90% Identität mit SEQ ID NO:3 oder SEQ ID NO:4 aufweist, erkennt;
und
wobei das Verfahren zudem umfasst: das Einführen eines Polynukleotids in eine Kieselalge, welches zumindest eine Region umfasst, die zu einem Teil des selektierbaren Marker-Gens homolog ist, so dass das Polynukleotid sich mit dem selektierbaren Marker-Gen verbindet.

2. Verfahren nach Anspruch 1, wobei die TALE-Nuklease eine Aminosäuresequenz aufweist, die aus der Gruppe bestehend aus SEQ ID NO:5 bis SEQ ID NO:8 ausgewählt ist oder mindestens 80% Identität mit einer aus SEQ ID NO:5 bis SEQ ID NO:8 ausgewählten Aminosäuresequenz aufweist.

3. Verfahren nach Anspruch 1, welches in Schritt c) das Transformieren der Kieselalge mit einem für eine TALE-Nuklease kodierenden Polynukleotid und das Exprimieren der TALE-Nuklease in die Zelle umfasst.

4. Verfahren nach Anspruch 1, wobei die Kieselalge ausgewählt ist aus der Gruppe bestehend aus *Thalassiosira pseudonana* oder *Phaeodactylum tricornutum*.

5. Verfahren nach einem der Ansprüche 1 bis 4, wobei das selektierbare Marker-Gen das Uridin-5'-Monophosphat-Synthase-Gen (UMPS-Gen) ist, und das toxische Substrat die 5-Fluororotsäure (5-FOA) ist.

6. Verfahren nach einem der Ansprüche 1 bis 4, wobei das selektierbare Marker-Gen das Nitrat-Reduktase-Gen ist, und das toxische Substrat Chlorat ist.

7. Verfahren nach einem der Ansprüche 1 bis 6, welches zudem das Einführen mindestens eines weiteren interessierenden Proteins in die Kieselalge umfasst.

8. Verfahren nach Anspruch 7, wobei das weitere interessierende Protein eine Nuklease ist, die fähig ist, eine interessierende Zielsequenz zu erkennen und zu schneiden.

9. Verfahren nach einem der Ansprüche 1 bis 8, wobei das Selektieren der transformierten Kieselalgen erfolgt, ohne dass ein Antibiotika-selektierbares Marker-Gen in das Genom eingebaut ist.

10. Kieselalge, welche durch ein Verfahren nach einem der Ansprüche 1 bis 9 erhältlich ist und eine TALE-Nuklease umfasst, die eine Zielsequenz innerhalb eines Gens erkennt, welches aus dem UMPS-Gen (SEQ ID NO:1) und dem Nitrat-Reduktase-Gen (SEQ ID NO:2) ausgewählt ist, wobei die TALE-Nuklease eine Aminosäuresequenz aufweist, die aus der Gruppe bestehend aus SEQ ID NO:5 bis SEQ ID NO:8 ausgewählt ist oder mindestens 80% Identität mit einer aus SEQ ID NO:5 bis SEQ ID NO:8 ausgewählten Aminosäuresequenz aufweist; und wobei die TALE-Nuklease eine Zielsequenz erkennt, die mindestens 90% Identität mit SEQ ID NO:3 oder SEQ ID NO:4 aufweist.

## Revendications

1. Procédé de modification d'une diatomée comprenant :
(a) la sélection d'un gène marqueur de sélection au sein du génome de diatomée, qui code pour une protéine rendant une cellule sensible à un substrat toxique ;
(b) la fourniture d'une nucléase TALE qui reconnaît spécifiquement et clive une séquence cible au sein dudit gène marqueur de sélection ;
(c) l'introduction de ladite nucléase TALE dans des diatomées de sorte que le clivage par ladite nucléase inactive ledit gène marqueur de sélection ;
(d) la mise en culture desdites diatomées avec ledit substrat toxique ; et
(e) la sélection des diatomées qui sont résistantes au substrat toxique,
dans lequel ledit gène marqueur de sélection et ledit substrat toxique sont choisis parmi :
- ledit gène marqueur de sélection est le gène de l'uridine-5'-monophosphate synthase (UMPS) et ledit substrat toxique est l'acide 5-fluoroorotique (5-FOA), et
- ledit gène marqueur de sélection est le gène de la nitrate réductase et ledit substrat toxique est le chlorate,
dans lequel ladite nucléase TALE reconnaît une séquence cible sélectionnée parmi SEQ ID NO: 3 et SEQ ID NO: 4, ou une séquence cible présentant au moins 90 % d'identité avec SEQ ID NO: 3 ou SEQ ID NO: 4 ; et
où ledit procédé comprend en outre : l'introduction dans ladite diatomée d'un polynucléotide comprenant au moins une région homologue à une partie dudit gène marqueur de sélection de sorte que ledit polynucléotide se recombine avec ledit gène marqueur de sélection.

2. Procédé selon la revendication 1, dans lequel ladite nucléase TALE possède une séquence d'acides aminés sélectionnée dans le groupe consistant en SEQ ID NO: 5 à SEQ ID NO: 8, ou présente au moins 80 % d'identité avec une séquence d'acides aminés sélectionnée parmi SEQ ID NO: 5 à SEQ ID NO: 8.

3. Procédé selon la revendication 1, comprenant, à l'étape c), la transformation de ladite diatomée avec un polynucléotide codant pour ladite nucléase TALE et l'expression de ladite nucléase TALE dans la cellule.

4. Procédé selon la revendication 1, dans lequel ladite diatomée est sélectionnée dans le groupe consistant en : *Thalassiosira pseudonana* ou *Phaeodactylum tricornutum.*

5. Procédé selon l'une quelconque des revendications 1 à 4, dans lequel ledit gène marqueur de sélection est le gène de l'uridine-5'-monophosphate synthase (UMPS) et ledit substrat toxique est l'acide 5-fluoroorotique (5-FOA).

6. Procédé selon l'une quelconque des revendications 1 à 4, dans lequel ledit gène marqueur de sélection est le gène de la nitrate réductase et ledit substrat toxique est le chlorate.

7. Procédé selon l'une quelconque des revendications 1 à 6, comprenant en outre l'introduction d'au moins une autre protéine d'intérêt dans ladite diatomée.

8. Procédé selon la revendication 7, dans lequel ladite autre protéine d'intérêt est une nucléase capable de reconnaître et de cliver une séquence cible d'intérêt.

9. Procédé selon l'une quelconque des revendications 1 à 8, dans lequel la sélection des diatomées transformées est réalisée sans gène marqueur de sélection par un antibiotique intégré dans le génome.

10. Diatomée pouvant être obtenue par un procédé selon l'une quelconque des revendications 1 à 9, comprenant une nucléase TALE qui reconnaît une séquence cible au sein d'un gène sélectionné parmi le gène de l'UMPS (SEQ ID NO: 1) et le gène de la nitrate réductase (SEQ ID NO: 2), dans laquelle la nucléase TALE possède une séquence d'acides aminés sélectionnée dans le groupe consistant en SEQ ID NO: 5 à SEQ ID NO: 8, ou présente au moins 80 % d'identité avec une séquence d'acides aminés sélectionnée parmi SEQ ID NO: 5 à SEQ ID NO: 8 ; et dans laquelle la nucléase TALE reconnaît une séquence cible présentant au moins 90 % d'identité avec SEQ ID NO: 3 ou SEQ ID NO: 4.
